# EUROPEAN PATENT APPLICATION

(11) **EP 2 474 550 A1**
(43) Date of publication of application: **11.07.2012**
(21) Application number: 10075763.2
(22) Date of filing: 22.12.2010
(51) Int. Cl.: C07D 493/08, A61P 35/00, A61K 31/35

(54) **Derivatives of Englerin for the treatment of cancer**

(71) Applicant: Max-Planck-Gesellschaft zur Förderung der Wissenschaften e.V., 80539 München (DE); Technische Universität Dortmund, 44227 Dortmund (DE)
(72) Inventor: Christmann, Mathias, Prof. Dr., 44229 Dortmund (DE); Radtke, Lea, 44139 Dortmund (DE); Waldmann, Herbert, Prof. Dr., 44265 Dortmund (DE); Willot, Matthieu, Dr., 44139 Dortmund (DE); Ziegler, Slava, Dr., 44269 Dortmund (DE); Sun, Hongyan, Dr., 44225 Dortmund (DE)
(74) Representative: Arth, Hans-Lothar

(57) **Abstract**

The present invention relates to compounds of the general formula (I) 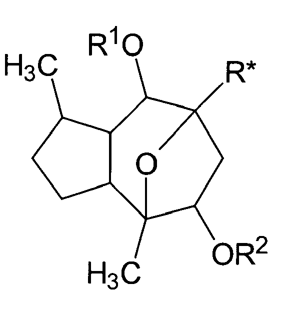 which show a specific activity against cancer cell lines, the use of these compounds for prophylaxis and treatment of cancer as well as to pharmaceutical compositions containing at least one compound of general formula (I).

## Description

The present invention relates to derivatives of Englerin A which show a specific activity against cancer and especially renal cancer, the use of these compounds for prophylaxis and treatment of cancer and especially renal cancer as well as to pharmaceutical compositions containing at least one of these Englerin A derivative.

### Background of the invention

Kidney cancer is a major cause of morbidity and mortality in adults. Currently available drugs do not produce complete responses and have serious adverse side effects. Thus, the search for new agents which display specific activity against renal cancers is of great interest.

Englerin is a guaiane sesquiterpene, which has been isolated and identified originally from the bark of *Phyllanthus engleri,* a plant naturally growing in East Africa, particularly Tanzania and Zimbabwe. The plant has been used in traditional medicine. The isolation of Englerins A and B from stem bark of *P*. *engleri* was first described by Ratnayake et al. (Org. Lett. 2009, 11(1), 57-60).

The compound Englerin A has been demonstrated to have specific activity against cancer, particularly renal cancer. There is always a desire to generate more effective compounds for the treatment of cancer and specific kinds of cancer. Consequently it is the objective of the present invention to provide more active compounds than the natural product Englerin A.

### Description

It was surprisingly found that certain compounds of general formula (A) are highly potent anti-cancer agents. Moreover it was surprisingly found that the compounds of general formula (A) are active against renal cancer cell lines, but not against non-mutated renal cell lines. It was further surprisingly found that some compounds of the general formula (A) have more than twice the potency of the natural compound. Furthermore it was surprisingly found that several compounds of general formula (A) have a potency exceeding that of the natural compound.

The invention relates particularly to preferred compounds having the general formula (1): 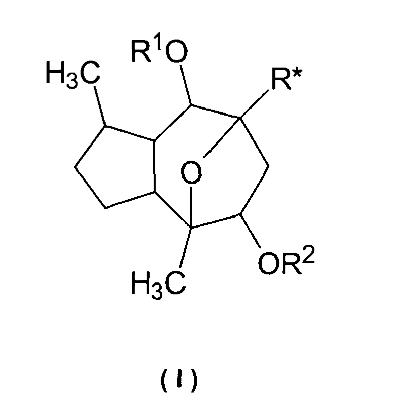 wherein
**R*** represents -CH₃, -C₂H₅, -C₃H₇, -CH(CH₃)₂, cyclo-C₃H₅, cyclo-C₄H₇, cyclo-C₅H₉, cyclo-C₆H₁₁, -Ph, -CH₂-Ph, -CH=CH-Ph;
**R¹** represents -CO-R³, -CO-CH=CH-R³, -CO-CH=C(CH₃)-R³;
**R²** represents -CO-CH₂-O-CO-NH-R⁴, -CO-CH₂-O-CO-N(R⁴R^{4*}), -CO-CH₂-OR⁴, -CO-CH₂-O-CO-R⁴, -CO-CH₂-O-CO-O-R⁴, -CO-CH₂-SR⁴, -CO-CH₂-N(R⁴R^{4*}), -CO-CH₂-NH-CO-O-R⁴ -CO-(CH₂)ₙ-OR⁴, -CO-(CH₂)ₙ-R⁴, 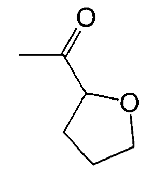 n is an integer selected from 1, 2, 3, 4, 5;
**R³** represents one of the following residues: 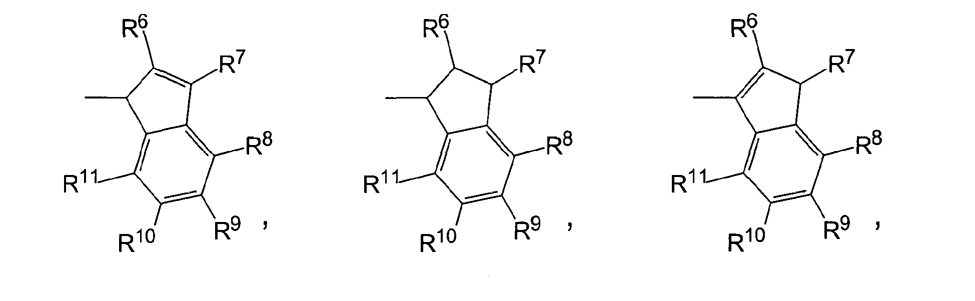 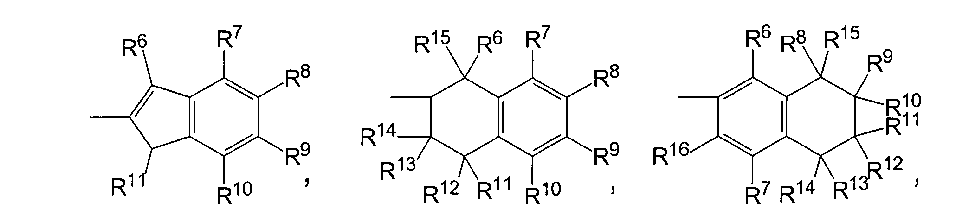 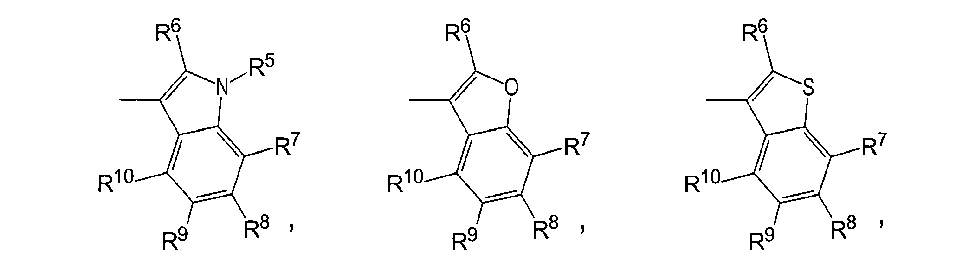 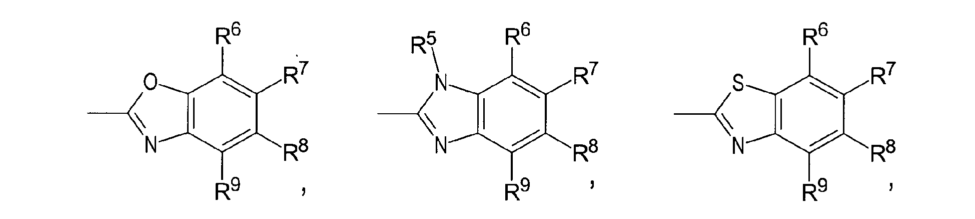 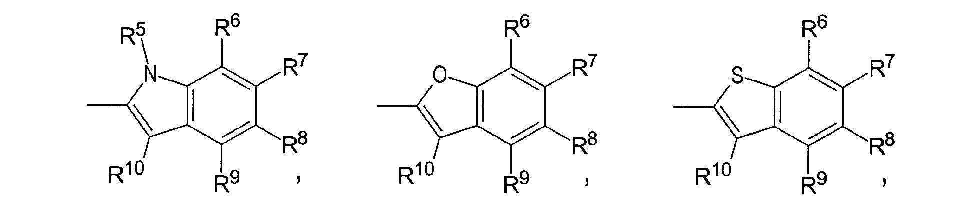 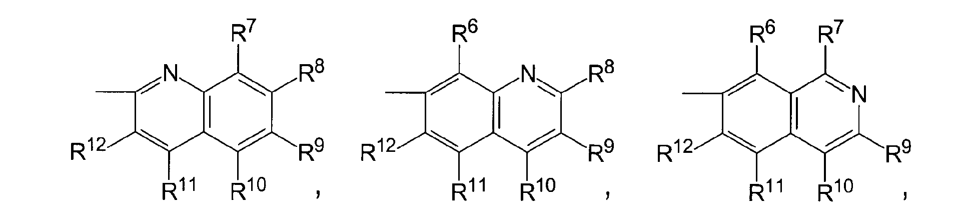 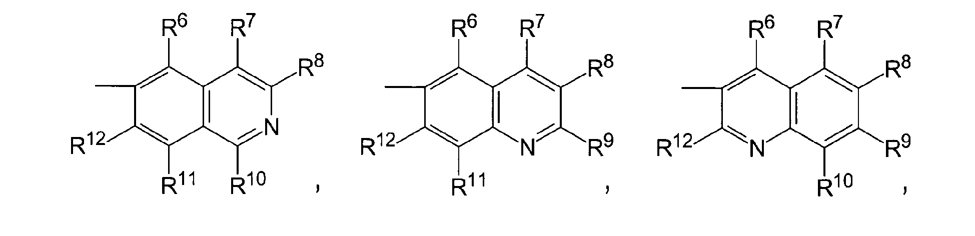 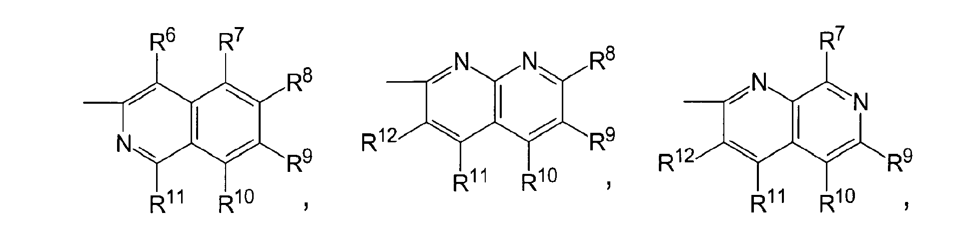 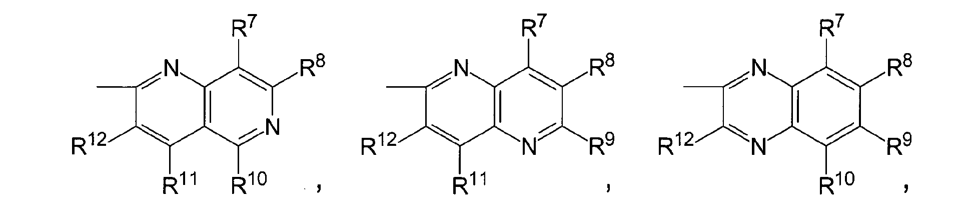 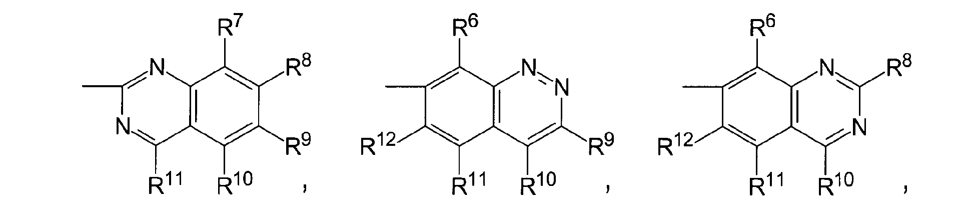 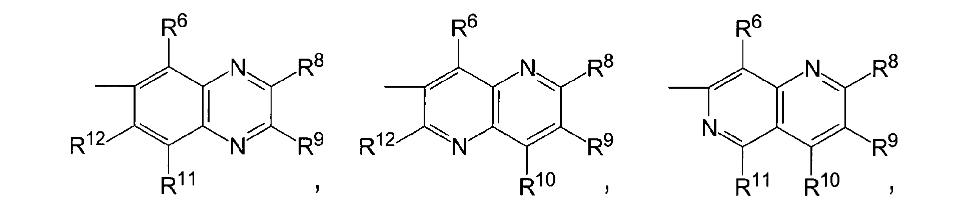 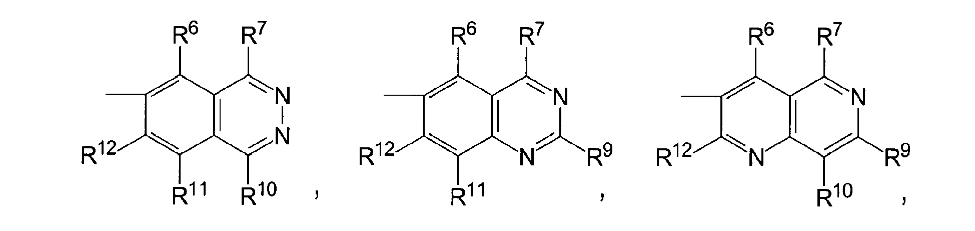 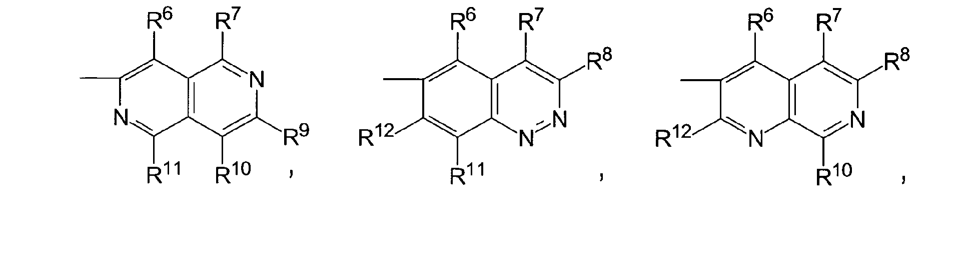 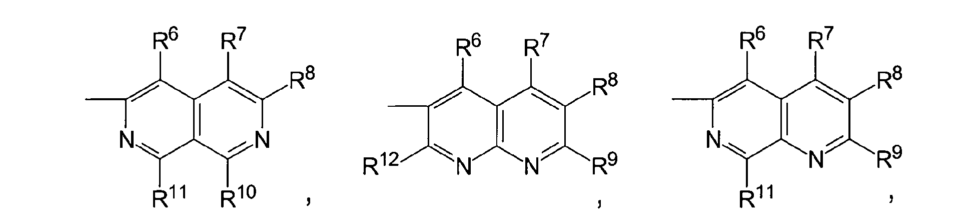 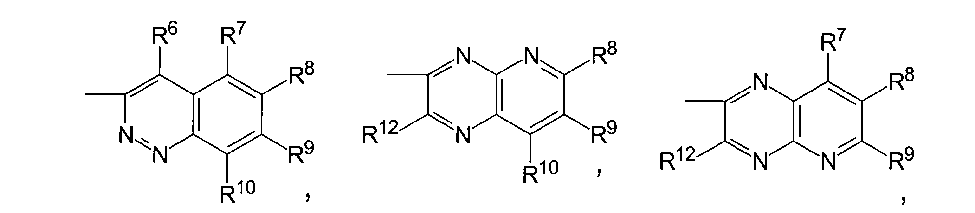 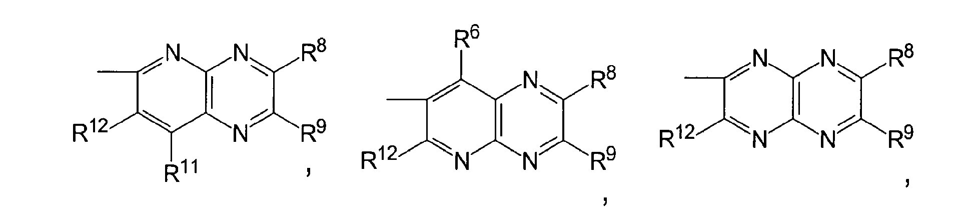 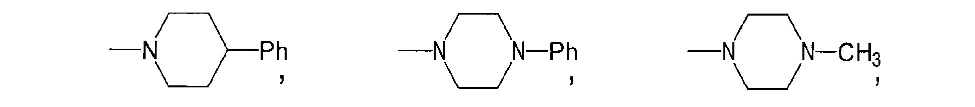 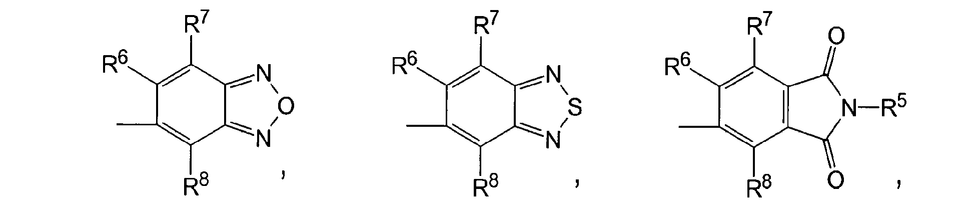 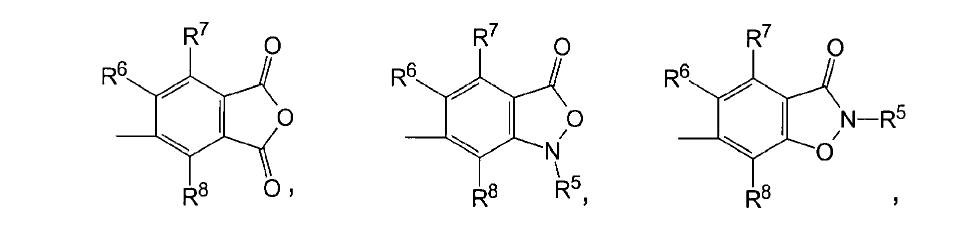 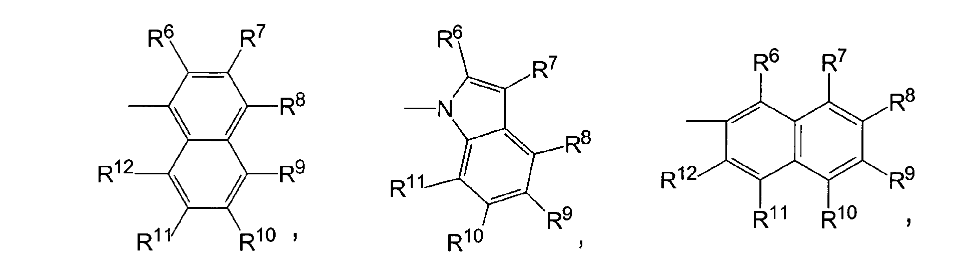 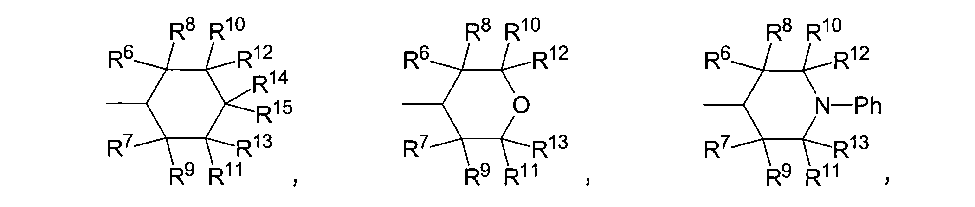 **R⁴, R^{4*}, R⁵** represent independently of each other **-R¹⁶, -R¹⁷, -R¹⁸**, -H, -CH₃, -C₂H₅, -C₃H₇, -CH(CH₃)₂, -C₄H₉, -C₅H₁₁, -CH₂-CH(CH₃)₂, -CH(CH₃)-C₂H₅, -C(CH₃)₃, -C₆H₁₃, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₉H₁₉, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH_{3,} -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH3)-_{C}(CH3)=CH₂, -C(CH₃)=CH-C(CH₃)=CH₂, -C(CH₃)=C(CH₃)-CH=CH₂. -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂Hₐ-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃;
**R⁶** to **R¹⁸** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] , -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃;
and epimers, enantiomers, stereoisomeric forms, mixtures of enantiomers, diastereomers, mixtures of diastereomers, hydrates, solvates and racemates of the above mentioned compounds and pharmaceutically acceptable salts thereof.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The compounds of the general formula (I) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (A) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Preferred are the compounds of general formula (A) and (B) 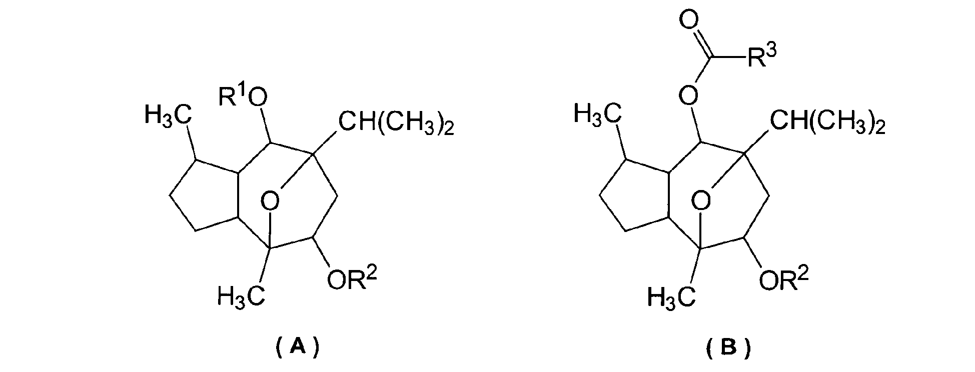 wherein
**R¹** and **R²** in formula (A) and wherein **R²** and **R³** in formula (B) have the meanings as disclosed herein.

Also preferred are the compounds of general formula (A) and (B) wherein R³ represents 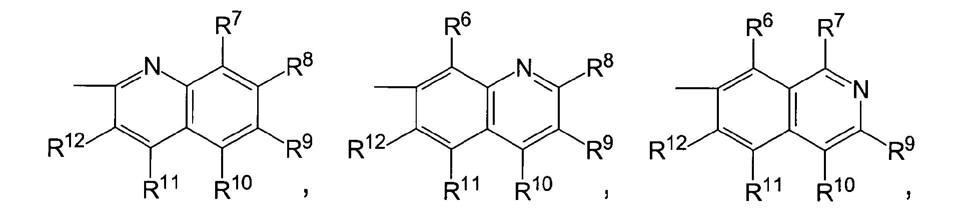 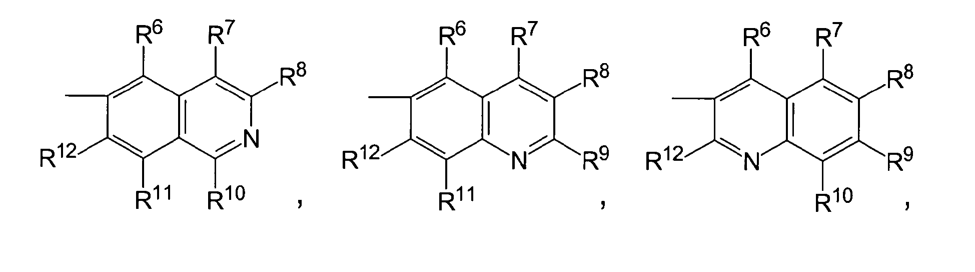 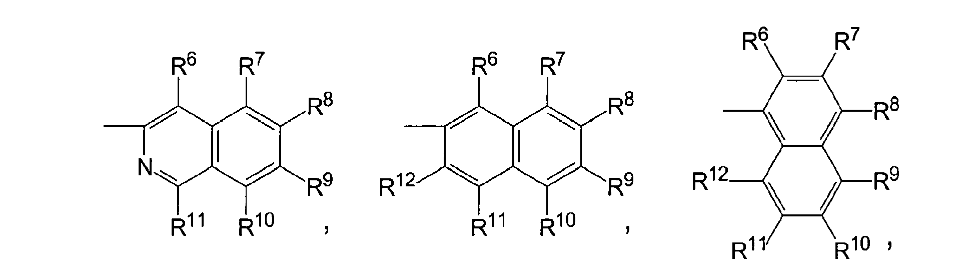 and wherein R⁶ to R¹² have the meanings as disclosed herein.

Preferred are also compounds of the general formula (AB) and (B), wherein R¹ represents -CO-CH=CH-R³ or -CO-CH=C(CH₃)-R³ and R³ represents 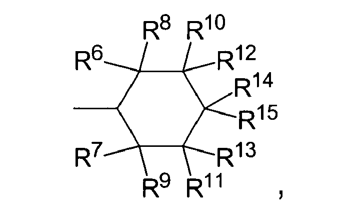 wherein R⁶ to R¹⁵ have the meanings as disclosed herein.

Moreover the compounds are preferred, wherein R² represents -CO-CH₂-OR⁴ and R⁴ represents -C₃H₇, -CH=CH₂, -CH₂-CH=CH₂, -CH=CH-CH₃, -C₂H₄-CH=CH₂, -CH₂-CH=CH-CH₃, -CH=CH-C₂H₅, -CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇ or -C≡C-C≡CH.

Furthermore it is preferred that the inventive compounds of formula (A) and formula (B) have the stereochemistry as shown in formula (C) 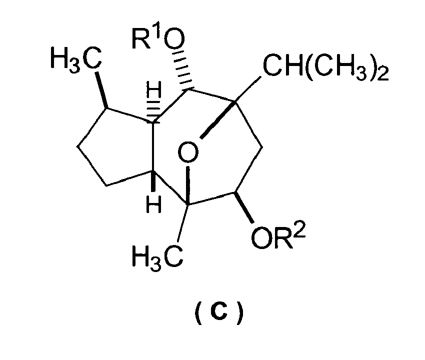 wherein R¹ and R² have the meanings as disclosed herein. Formula (C) shows the stereochemistry which is preferred for all inventive compounds. If this stereochemistry is transferred to general formula (B) the following formula (D) will be obtained, which shows the preferred stereochemistry.

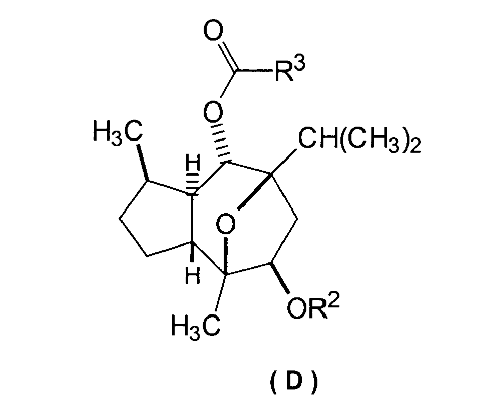

The inventive compounds are particularly useful for use in medicine, because they can be used for selective treatment and/or prophylaxis of cancer with minimal side effects on healthy cells. High levels of activity for in vitro and in vivo testing have been observed using the compounds of the present invention. This may lead to reduced dosages as compared with conventional therapeutics. Therefore the use of the compounds for treatment of cancer is preferred. Since the inventive compounds were never used as pharmaceutically active agent, another aspect of the present invention related to the inventive compounds for use as medicine.

The term "cancer" as used herein refers also to tumors, proliferative diseases, malignancies and their metastases. Cancers to be treated can be selected from the group consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer, without being limited to these diseases.

The inventive compound are preferably used for the treatment of breast cancer, ovarian cancer, liver cancer, leukemia, colon cancer, melanoma, prostate cancer, renal cancer, and lung cancer, wherein the treatment of renal cancer is particularly preferred.

The term treatment as used herein can mean the complete eradication of a cancer of can refer to partial tumour regression, to the inhibition of tumour growth, i.e. arrest of progression of the cancer, and to inhibition of metastasis. Consequently, the inventive compounds are also useful in palliative medicine. In other words, the term "a therapeutically effective amount" as used herein means a sufficient amount of the peptide of the invention to produce a therapeutic effect, as defined above, in a subject or patient in need of treatment.

The term prophylaxis as used herein is used to define an application of the inventive compounds to prevent re-growth of the tumour after surgical removal of a tumour or after a successful chemotherapeutic treatment. Prophylaxis as used herein can also mean to prevent metastasis or the development of cancers in people at particular risk.

A measure for the efficacy of a compound is IC50, which is the half maximal inhibitory concentration of a compound in inhibiting biological or biochemical function. This indicates how much of a particular drug or other substance is necessary to inhibit a biological process or a component of a process, such as an enzyme or a cell. It is commonly as a measure of antagonist drug potency in pharmacological research. In other words, it is the concentration of a drug which is required for 50% inhibition in vitro.

A closely related measure for the inhibitory effectivity of a compound is the GI50 value, which is the concentration required to achieve 50% growth inhibition. Both IC50 and GI50 can be expressed in nM or µM.

The inventive compounds have when administered an IC₅₀ of preferably less than 1,5 µM, more preferably less than 1 µM, more preferably less than 500 µM, more preferably less than 100 nM, more preferably less than 50 nM, more preferably less than 35 nM, more preferably less than 25 nM, more preferably below 20 nM and most preferably below 16 nM. The activity was determined as described in the examples.

The compounds of the invention are useful for the manufacture of a medicament for the treatment of cancer. Consequently another aspect of the present invention relates to the use of the compounds of general formula (I) for the manufacture of a pharmaceutical composition for the treatment of cancer, tumors, metastases and proliferative diseases. A medicament as used herein is a pharmaceutical drug for use in the treatment or prevention of cancer, tumors, metastases and proliferative diseases. It comprises particularly forms of the drug for application to an animal or human being. Thus, the drug is administered to the animal or patient together with at least one pharmaceutically acceptable carrier, cryprotectant, lyoprotectant, excipient and/or diluents. Preferably the pharmaceutical composition is further present in form of a lyophilisate or liquid buffer solution.

The compounds of the general formula (I) can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. The preferred preparations and formulations are in administratable form which is suitable for oral application. These administratable forms, for example, include pills, tablets, film tablets, coated tablets, capsules, powders and deposits. Other than oral administratable forms are also possible. The inventive compounds of general formula (I) or pharmaceutical preparations or formulations containing said compounds may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

Within the disclosed methods the pharmaceutical compositions of the present invention, containing at least one inventive compound of the general formula (I) or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophilic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the inventive compounds of the present invention may be found in "Remington's Pharmaceutical Sciences" Mack Publishing Co., Easton PA. A suitable composition comprising at least one compound of the invention and/or pharmaceutically acceptable salts thereof may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of the general formula (I) refers to that amount of the compound that results in an at least partial inhibition of cell growth. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. More preferably, the dosage of the compound corresponds to an effective concentration in the range of 1 nM to 5 µM. The actual amount of the composition administered will be dependent on the subject being treated, on the subject's weight, the severity of the affliction, the manner of administration and the judgement of the prescribing physician.

The present invention is further related to a method of treating and preventing cancer, especially renal cancer in a mammal comprising administering to said mammal an effective amount of at least one inventive compound. The term mammals include human patients and non-human primates, as well as experimental animals such as rabbits, rats, and mice, and other animals. The treatment of human patients is preferred.

The inventive compounds can be used for the treatment and/or prophylaxis of cancer in combination administration with another therapeutic compound or anti-cancer agent. However, excluded from the scope of the present application are the following compounds: Englerin A, Englerin B, 2'-Chloroenglerin A, 2'-Chloro,3'-hydroxydihydroenglerin A (epimer 1), 2'-Chloro,3'-hydroxydihydroenglerin A (epimer 2), 2',3'-Dichlorodihydroenglerin A (epimer 1), 2',3'-Dichlorodihydroenglerin A (epimer 2), 2'-Chloro,3'-ethoxydihydroenglerin A, 2'Chloro,3'-hydroxydihydroenglerin A (epimer 3), 2'Chloro,3'-hydroxydihydroenglerin A (epimer 4). As used herein the term "combination administration" of a compound, therapeutic agent or known drug with a compound of the present invention means administration of the drug and the inventive compound at such time that both the known drug and the inventive compound will have a therapeutic effect. In some cases this therapeutic effect will be synergistic. Such concomitant administration can involve concurrent (i.e. at the same time), prior, or subsequent administration of the drug with respect to the administration of the compound of the present invention. A person of ordinary skill in the art would have no difficulty determining the appropriate timing, sequence and dosages of administration for particular drugs and compounds of the present invention. Excluded by disclaimer from the present scope of protection are the compounds wherein the substituent R³ comprises an unsubstituted phenyl group.

The invention is further related to synthesis of the inventive compounds. The inventive compounds can be prepared by any synthesis methodology known in the chemical art. The synthesis of selected compounds is disclosed in the experimental part.

### General synthesis of the inventive compounds

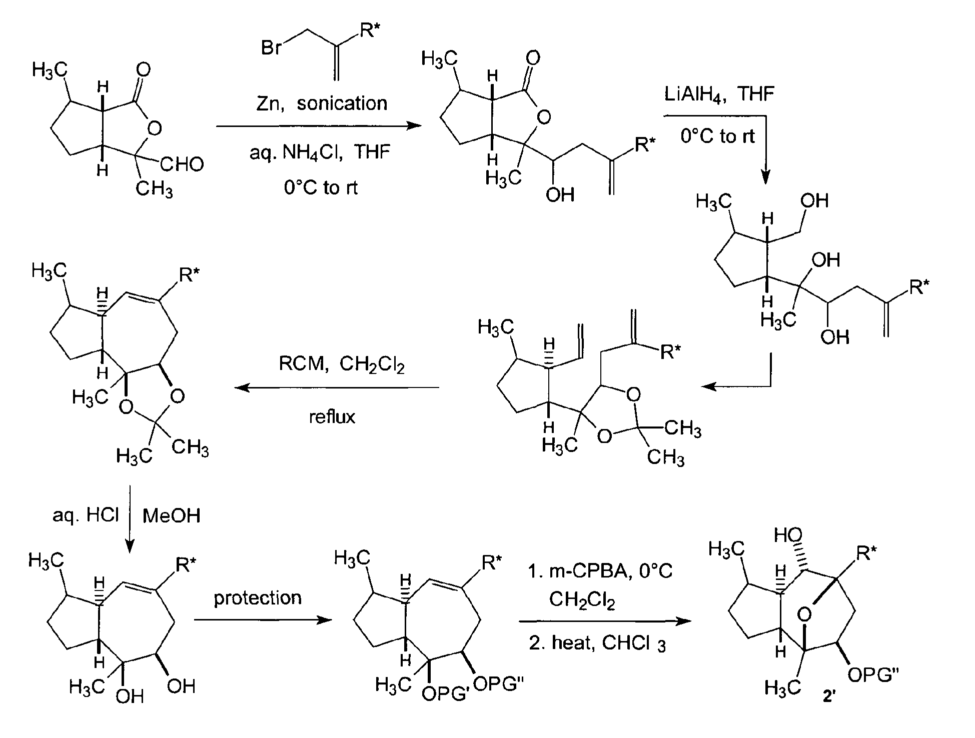

As starting material nepetalactone might be used or a nepetalactone isomer which is converted to the aldehyde and which is than subjected to Barbier-type allylation in order to obtain the allyl lactone compound. The residue R* is introduced into the inventive compounds through the corresponding allyl halogenide and preferably the corresponding allyl bromide. It is at this stage possible to recycle the minor isomer. Reduction of the allyl lactone with LiAlH₄ results in the corresponding triols in excellent yields. Preferably the LiAlH₄ reduction step is a diastereoselective concomitant reduction of both the ketone and the lactone moiety. The obtained triols are in most cases crystalline. These triols were converted to the corresponding dienes as precursors for the crucial ring closing metathesis reaction. Thereafter the ketal is cleaved under acidic conditions and the obtained vicinal diol is protected using the protecting groups PG' and PG". To improve the moderate diastereoselectivity for the olefin epoxidation with m-CPBA (m-Chloroperbenzoic acid) the monoprotected diol was used, wherein PG' is hydrogen and PG" is a protecting group preferably for secondary alcohols and more preferably a silyl protecting group such as trimethylsilyl (TMS), tert-butyldiphenylsilyl (TBDPS), tert-butyldimethylsilyl (TBS or TBDMS), triisopropylsilyl (TIPS) and [2-(trimethylsilyl)ethoxy]methyl (SEM) and most preferably TBS. The PG" protected tricyclic key intermediate 2' is obtained after heat induced transannular epoxide opening to form the highly crystalline compound 2' having the stereochemistry as shown above. The compound 2 is the corresponding key intermediate wherein the stereochemistry is not indicated and consequently covers all stereoisomeric forms of the key intermediate 2'.

Subsequently, different ester side chains indicated as R¹ and R² were introduced by common esterification reactions in order to obtain the inventive compounds (I').

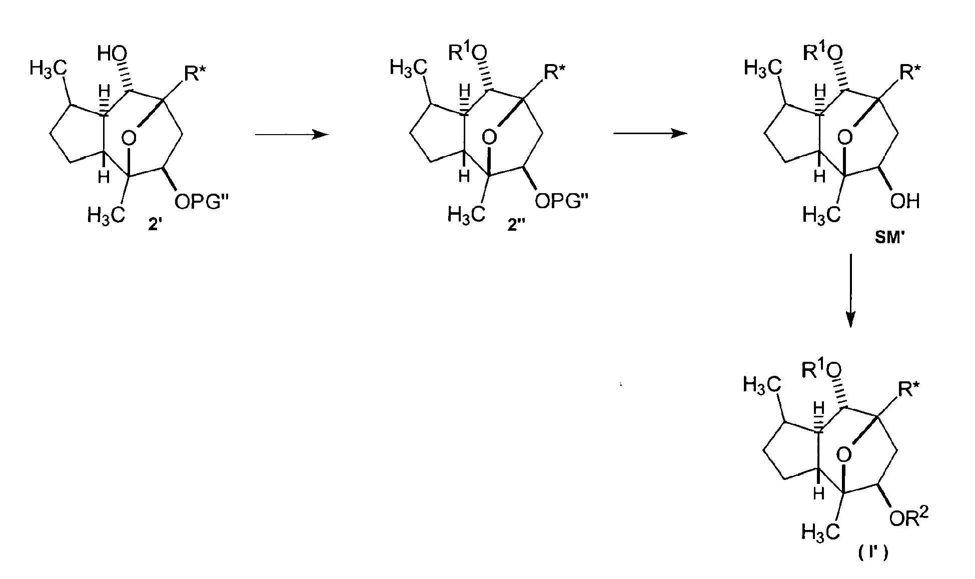

The substituents R¹, R² and R* have the meanings as disclosed in claim 1 of the present application.

### EXAMPLES

Modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims. Variations or amendments of the invention which are obvious to an expert fall within the scope of protection as displayed by the claims.

### GENERAL PROCEDURE

Solvents of HPLC grade were purchased from Fisher Scientific or VWR (Prolabo). Where dry solvents (Et₂O, CH₂Cl₂, toluene, DMF or THF) were required, they were purified by Solvent Purification Systems M-BRAUN Glovebox Technology SPS-800. Technical quality solvents for column chromatography were used after short path distillation in a rotary evaporator. Unless noted below, all other compounds were reported in literature or were supplied by Aldrich, Acros, ABCR or AlfaAesar and used without further purification. *Thin-layer chromatography* (SiO₂, *TLC*) was performed on Merck TLC silica gel 60 F₂₅₄. *Column chromatography* was performed on Merck silica gel 60 (0.040 - 0.063 nm), using standard flash chromatographic methods. *Optical rotations* were recorded on a Perkin Elmer polarimeter 341 at 589 nm and were reported as [α]_{D} (concentration). *NMR spectra* were recorded on Bruker DRX300 (300 MHz), DRX400 (400 MHz), DRX500 (500 MHz) or DRX600 (600 MHz) spectrometers and were referenced against the residual solvent peaks [CHCl₃: δ 7.26 ppm (¹H NMR) and 77.16 ppm (¹³C NMR), CD₃OD: δ 3.31 ppm (¹H NMR) and 49.00 ppm (¹³C NMR)]. Chemical shifts are reported in parts per million as follows: chemical shift, multiplicity (s = singlet, d = doublet, t = triplet, q = quartet, m = multiplet, br = broad), coupling constant, and integration. *Infrared spectra* were recorded on a Nicolet Impact 400D spectrometer on potassium bromide matrix (disk or film). *Low resolution mass spectra* were performed on a Thermo TSQ mass spectrometer, Hewlett Packard 6890 series/Mass selective detector. *High resolution mass spectra* were recorded on a LTQ Orbitrap mass spectrometer coupled to an Accela HPLC-System (HPLC column: Hypersyl GOLD, 50 mm × 1 mm, 1.9 µm). All instruments are from Thermo Electron.

### SYNTHESIS OF COMPOUNDS

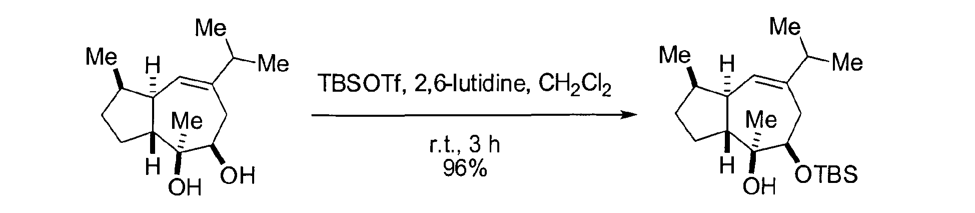

To a stirred solution of the diol (1.56 g, 6.54 mmol, 1 equiv.) in dry CH₂Cl₂ (32.7 mL) under argon atmosphere were added successively freshly distilled 2,6-lutidine (1.83 mL, 15.7 mmol, 2.4 equiv.) and *tert*-butyldimethylsilyl trifluoromethanesulfonate (1.80 mL, 7.85 mmol, 1.2 equiv.). After 3 h of stirring at rt, the solution was quenched with H₂O and extracted twice with CH₂Cl₂. The organic phases were washed with brine, dried over MgSO₄, and concentrated under reduced pressure after filtration. The residue was purified by flash chromatography (SiO₂, c-Hexane/AcOEt 50:1 to 20:1) to afford the TBS-protected compound (2.21 g, 6.27 mmol, 96%) as colorless oil: [α]_{D}²⁰ +5.5° (c 0.94, CHCl₃). ¹H NMR (400 MHz, CDCl₃) δ 5.38 (m, 1H), 3.54 (dd, *J* = 10.5, 1.5 Hz, 1H), 2.91 (brs, 1H), 2.68 (dddd, *J* = 16.8, 10.5, 3.0, 2.5 Hz, 1H), 2.26-2.09 (m, 4H), 1.84 (m, 1H), 1.75 (d, *J* = 16.8 Hz, 1H), 1.71 (m, 1H), 1.59 (m, 1H), 1.35 (m, 1H), 1.11 (s, 3H), 0.98 (d, *J* = 7.0 Hz, 3H), 0.96 (d, *J* = 7.0 Hz, 3H), 0.91 (s, 9H), 0.78 (d, *J* = 7.0 Hz, 3H), 0.11 (s, 3H), 0.08 (s, 3H). ¹³C NMR (100 MHz, CDCl₃) δ 144.0, 124.8, 77.4, 74.9, 46.3, 43.7, 38.1, 37.6, 33.4, 33.3, 26.0 (3C), 24.2, 22.1, 21.6, 20.3, 18.1, 15.3, -3.7, -4.7. IR (KBr film) v (cm⁻¹) 3551, 2957. HPLC-ESI-HRMS ([M+Na]⁺) calcd for C₂₁H₄₀O₂SiNa 375.2690, found 375.2690; ([M+H]⁺) calcd for C₂₁H₄₁O₂Si 353.2870, found 353.2871.

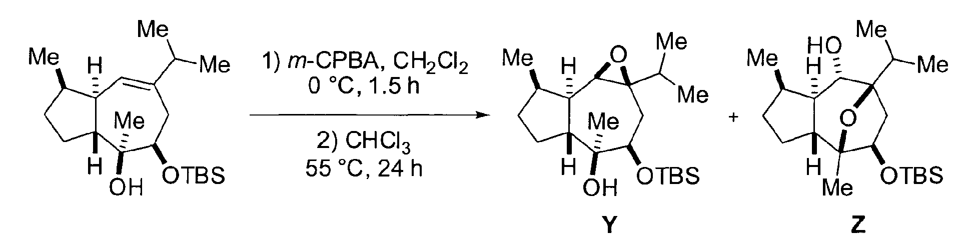

To a stirred solution of the TBS-protected compound (866.0 mg, 2.46 mmol, 1 equiv.) in CH₂Cl₂ (12.3 mL) under argon atmosphere at 0°C was added *m*-CPBA (70%, 726.5 mg, 2.95 mmol, 1.2 equiv.). After 1.5 h of stirring at 0°C, the solution was quenched with an aqueous KOH solution (10 wt%, 12.3 mL). The aqueous phase was extracted 3 times with CH₂Cl₂. The combined organic phases were dried over MgSO₄ and concentrated under reduced pressure after filtration. The residue was dissolved in CHCl₃ (12.3 mL) and heated to 55°C. After 24 h of stirring at 55°C, the solution was concentrated under reduced pressure. The residue was purified by flash chromatography (SiO₂, *c*-Hexane/AcOEt 20:1 to 10:1) to afford the epoxy compound **Y** (131.3 mg, 356 µmol, 15%) as colorless oil and **Z** (761.2 mg, 2.06 mmol, 84%) as white solid: [α]_{D}²⁰ -59.7° (*c* 0.87, CHCl₃). ¹H NMR (300 MHz, CDCl₃) δ 3.89 (dd, *J* = 7.3, 2.6 Hz, 1H), 3.61 (d, *J* = 10.2 Hz, 1H), 2.30 (dd, *J* = 13.9, 7.3 Hz, 1H), 2.30 (m, 1H), 2.01 (m, 1H), 1.96 (septet, *J* = 7.0 Hz, 1H), 1.66 (m, 1H), 1.61 (dd, *J* = 13.9, 2.6 Hz, 1H), 1.56 (m, 1H), 1.29-1.12 (m, 3H), 1.16 (s, 3H), 1.05 (d, *J* = 7.0 Hz, 6H), 0.89 (s, 9H), 0.88 (d, *J* = 7.0 Hz, 3H), 0.04 (s, 3H), 0.02 (s, 3H). ¹³C NMR (100 MHz, CDCl₃) δ 85.6, 85.6, 73.1, 71.1, 47.9, 42.4, 32.4, 31.5, 30.6, 27.1, 26.1, 25.9 (3C), 19.8, 18.5, 18.3, 17.6, 17.2, -4.5, -4.9. IR (KBr film) v (cm⁻¹) 3408, 3381, 2946, 2861. HPLC-ESI-HRMS ([M+H]⁺) calcd for C₂₁H₄₁O₃Si 369.2819, found 369.2820.

### Variation of the cinnamic ester

### General procedure for the synthesis of compound 45

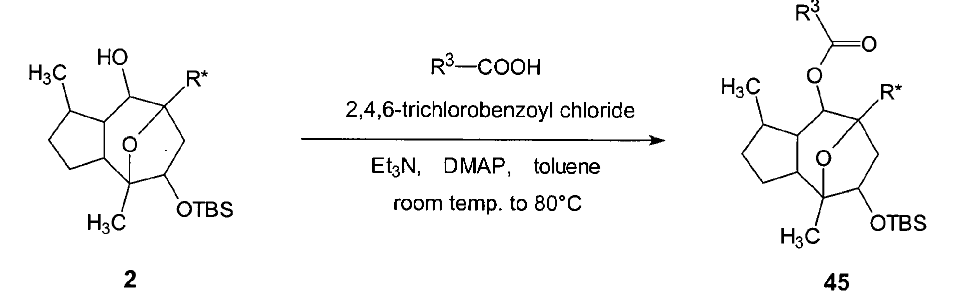

To a stirred solution of acid (R³COOH, 2 equiv.) solved in dry toluene (0.1 M) under argon atmosphere were added successively Et₃N (4 equiv.) and 2,4,6-trichlorobenzoyl chloride (2.2 equiv.). After 1 h of stirring at room temperature (rt), alcohol **2** (1 equiv.) and DMAP (2.6 equiv.) were added and the solution was heated to 80°C. After 3 to 23 h of stirring at 80°C, the solution was quenched with an aqueous saturated NaHCO₃ solution. The phases were separated and the aqueous phase was extracted twice with ethyl acetate. The combined organic phases were washed with brine, dried over MgSO₄ and concentrated under reduced pressure after filtration. The residue was purified by flash chromatography (SiO₂, c-Hexane/AcOEt 50:1 to 20:1) to afford compounds **45**.

### General procedure for the synthesis of compound 46

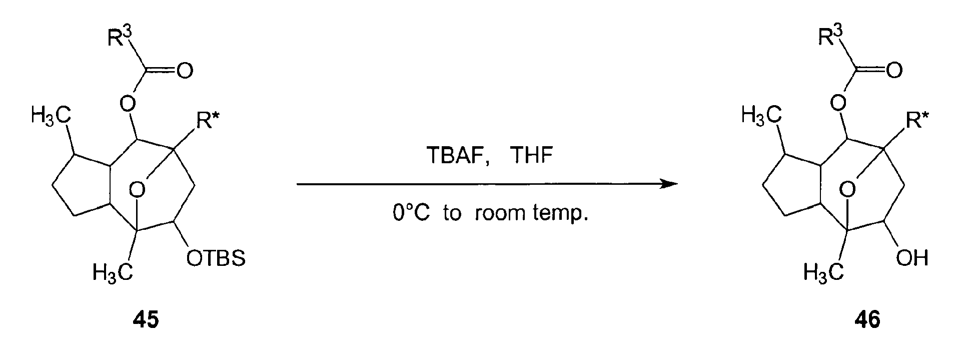

To a stirred solution of compound **45** (1 equiv.) in dry THF (0.02 M) under argon atmosphere was added TBAF (1 M in THF, 2 equiv.) at 0°C. After 2 to 24 h of stirring at rt, the solution was quenched with an aqueous saturated NH₄Cl solution. The phases were separated and the aqueous phase was extracted twice with ethyl acetate. The combined organic phases were washed with brine, dried over MgSO₄ and concentrated under reduced pressure after filtration. The residue was purified by flash chromatography (SiO₂, c-Hexane/AcOEt 5:1 to 2:1) to afford compound **46**.

### General procedure for the synthesis of compound 47

### Method A

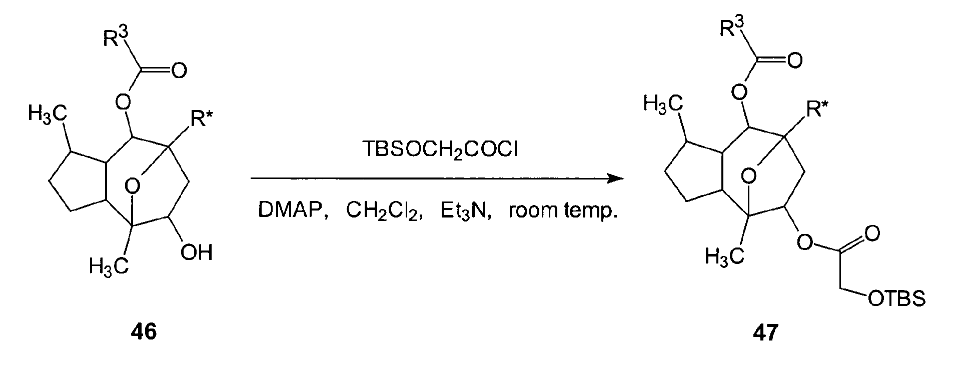

To a stirred solution of compound **46** (1 equiv.) in a 2:1 mixture of dry CH₂Cl₂/Et₃N (0.155 M) under argon atmosphere were added successively DMAP (3 equiv.) and 2-((*tert*-butyldimethylsilyl)oxy)acetyl chloride (4 equiv.) at rt. After 5 to 24 h of stirring at rt, the solvent was evaporated and the residue was purified by flash chromatography (SiO₂, *c*-Hexane/AcOEt 10:1 to 5:1) to afford compound **47**.

### Method B

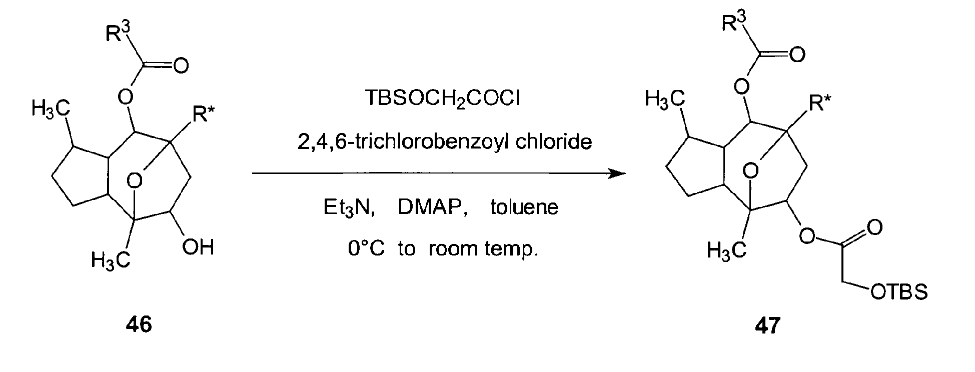

To a stirred solution of compound **46** (1 equiv.) in dry toluene were added at 0°C under argon atmosphere successively 2-((*tert*-butyldimethylsilyl)oxy)acetic acid (2 equiv.), DMAP (2.6 equiv.), 2,4,6-trichlorobenzoyl chloride (2.2 equiv.) and Et₃N (4 equiv.). After 24 h of stirring at rt, the solution was quenched with an aqueous saturated NaHCO₃ solution. The phases were separated and the aqueous phase was extracted twice with ethyl acetate. The combined organic phases were washed with brine, dried over MgSO₄ and concentrated under reduced pressure after filtration. The residue was purified by flash chromatography (SiO₂, c-Hexane/AcOEt 10:1 to 2:1) to afford compound **47**.

### General procedure for the synthesis of compound 48

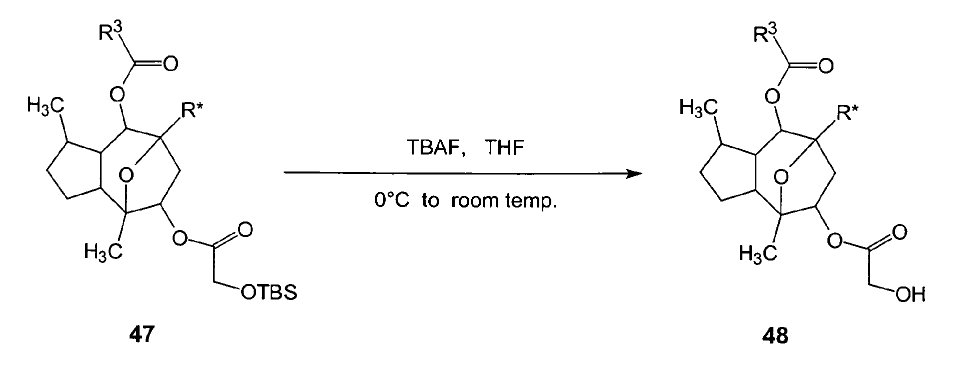

To a stirred solution of compound **47** (1 equiv.) in dry THF (0.02 M) under argon atmosphere at 0°C was added TBAF (1 M in THF, 2 equiv.). After 2 to 24 h of stirring at rt, the solution was quenched with an aqueous saturated NH₄Cl solution. The phases were separated and the aqueous phase was extracted twice with ethyl acetate. The combined organic phases were washed with brine, dried over MgSO₄ and concentrated under reduced pressure after filtration. The residue was purified by flash chromatography (SiO₂, c-Hexane/AcOEt 5:1 to 2:1) to afford compound **48**.

### Variation of the glycolic ester

### General procedure for the synthesis of compounds 36-43

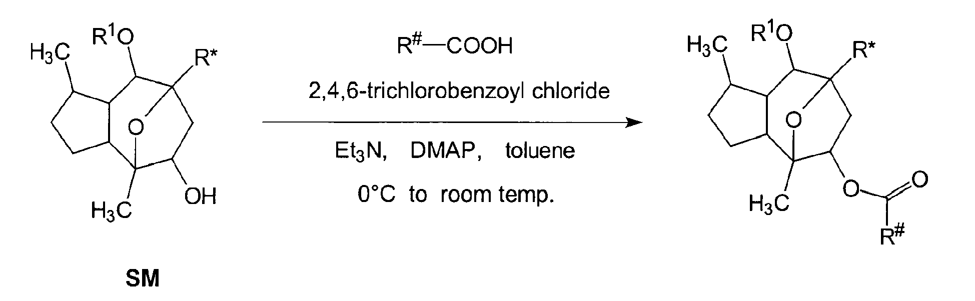

R^{#} represents -CH₂-O-CO-NH-R⁴, -CH₂-O-CO-N(R⁴R^{4*}), -CH₂-O-CO-O-R⁴, -CH₂-OR⁴, -CH₂-O-CO-R⁴, -CH₂-SR⁴, -CH₂-N(R⁴R^{4*}), -CH₂-NH-CO-O-R⁴ -(CH₂)ₙ-OR⁴, -(CH₂)ₙ-R⁴, 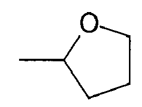 and n is an integer selected from 1, 2, 3, 4, 5.

To a stirred solution of the alcohol **SM** in dry toluene (0.026 M) under argon atmosphere at 0°C were added successively acid (R^{#}COOH, 2 equiv.), Et₃N (4 equiv.), DMAP (2.6 equiv.) and 2,4,6-trichlorobenzoyl chloride (2.2 equiv). After 2 to 3 h of stirring at rt, the reaction was quenched with an aqueous saturated NaHCO₃ solution. The phases were separated and the aqueous phase was extracted twice with ethyl acetate. The combined organic phases were dried over MgSO₄ and concentrated under reduced pressure after filtration. Thereafter the residue was purified by flash chromatography (SiO₂, c-Hexane/AcOEt 5:1 to 2:1) and various acyl compounds were obtained.

The compounds **36-43** were obtained by this method.

### ANALYSIS OF THE ANALOGUES 13 & 14

| | |
|---|---|
| **13** | [α]_{D}²⁰ -33.1° (*c* 0.31, CHCl₃). ¹H NMR (400 MHz, CDCl₃) δ 7.67 (d, *J* = 16.1 Hz, 1H), 7.53 (m, 2H), 7.39 (m, 3H), 6.41 (d, *J* = 16.1 Hz,1H), 5.22 (dd, *J* = 7.8, 3.0 Hz, 1H), 5.00 (d, *J* = 10.5 Hz, 1H), 4.20 (s,2H), 2.65 (dd, *J =* 14.6, 8.0 Hz, 1H), 2.39 (brs, 1H), 2.18 (sextet, *J* =7.0 Hz, 1H), 1.98 (m, 1H), 1.84-1.64 (m, 4H), 1.59 (m, 2H), 1.26 (m,2H), 1.23 (s, 3H), 0.97 (t, *J* = 7.3 Hz, 3H), 0.91 (d, *J* = 7.3 Hz, 3H).¹³C NMR (100 MHz, CDCl₃) δ 173.2, 166.0, 145.4, 134.4, 130.6,129.1 (2C), 128.3 (2C), 117.9, 84.8, 83.9, 76.6, 72.4, 60.8, 47.9, 46.4,40.6, 31.2, 31.1, 27.5, 25.1, 19.1, 17.2, 7.8. IR (KBr film) (cm⁻¹) v3444, 2959, 2871, 1747, 1713, 1635. HPLC-ESI-HRMS ([M+H]⁺)calcd for C₂₅H₃₃O₆ 429.2272, found 429.2267. |
| 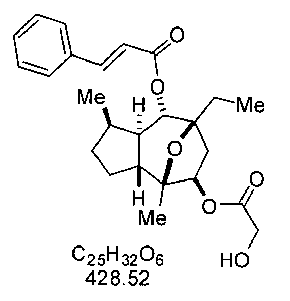 | |
| **14** | [α]_{D}²⁰ -41.9° (*c* 0.91, CHCl₃). ¹H NMR (500 MHz, CDCl₃) δ 7.68 (d, *J* = 16.1 Hz, 1H), 7.53 (dd, *J* = 6.4, 3.1 Hz, 2H), 7.40-7.39 (m, 3H),6.42 (d, *J* = 15.8 Hz, 1H), 5.21 (dd, *J* = 7.8, 3.3 Hz, 1H), 4.94 (d, *J* =10.5 Hz, 1H), 4.20 (s, 2H), 2.76 (dd, *J* = 14.3, 7.8 Hz, 1H), 2.39 (brs,1H), 2.20 (sextet, *J* = 6.8 Hz, 1H), 2.03-1.94 (m, 1H), 1.85-1.73 (m,2H), 1.62-1.52 (m, 3H), 1.34 (s, 3H), 1.30-1.27 (m, 1H), 1.24 (s, 3H),0.89 (d, *J* = 7.3 Hz, 3H). ¹³C NMR (100 MHz, CDCl₃) δ 173.0, 166.0,145.3, 134.2, 130.4, 128.9 (2C), 128.1 (2C), 117.7, 84.8, 81.6, 76.6,73.8, 60.6, 47.9, 46.1, 42.9, 31.0, 30.9, 25.2, 22.3, 18.9, 17.1. IR (KBrfilm) (cm⁻¹) v 3446, 2960, 2874, 2251, 1713. HPLC-ESI-HRMS([M+H]⁺) calcd for C₂₄H₃₁O₆ 415.2115, found 415.2114; HPLC-ESI-HRMS ([M+Na]⁺) calcd for C₂₄H₃₀O₆Na 437.1935, found 437.1932. |
| 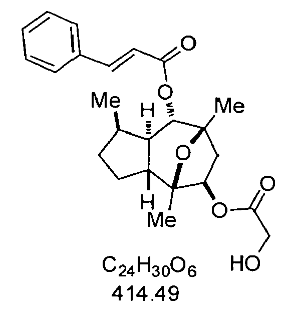 | |

### ANALYSIS OF THE COMPOUNDS (1) and 15-35

| | |
|---|---|
| (-) englerin A (1) | [α]_{D}²⁰ -31.4° (*c* 1.16, MeOH). ¹H NMR (600 MHz, CD₃OD) δ 7.69 (d, *J* = 16.1 Hz, 1H), 7.61 (m, 2H), 7.41 (brdd, *J* = 3.4, 3.2 Hz, 3H),6. 51 (d, *J =* 16.1 Hz, 1H), 5.26 (dd, *J* = 8.1, 3.2 Hz, 1H), 5.12 (d, *J* =10.3 Hz, 1H), 4.16 (brs, 2H), 2.70 (ddd, *J* = 14.7, 8.1, 1.2 Hz, 1H),2.13 (sextet, *J* = 6.8 Hz, 1H), 2.00 (m, 1H), 1.87 (m, 1H), 1.86 (dd,*J* = 14.7, 3.2 Hz, 1H), 1.75 (m, 2H), 1.67 (m, 1H), 1.36-1.22 (m, 2H),1.19 (s, 3H), 1.02 (d, *J* = 6.8 Hz, 3H), 0.97 (d, *J* = 7.1 Hz, 3H), 0.93(d, *J* = 7.1 Hz, 3H). ¹³C NMR (150 MHz, CD₃OD) δ 174.0, 167.3,146.8, 135.7, 131.6, 130.1 (2C), 129.3 (2C), 118.8, 86.7, 86.0, 76.6,72.5, 61.1, 48.9, 48.0, 40.7, 34.1, 32.5, 32.0, 25.5, 19.2, 18.6, 17.7,17.2. IR (KBr film) (cm⁻¹) ν 3471, 2961, 1712, 1635. HPLC-ESI-HRMS ([M+H]⁺) calcd for C₂₆H₃₅O₆ 443.2428, found 443.2427. |
| 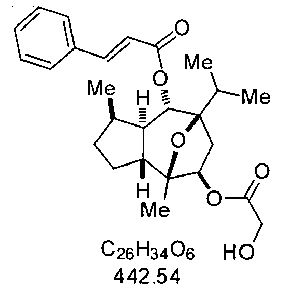 | |
| **15** | [α]_{D}²⁰ -31.8° (*c* 1.27, CHCl₃). ¹H NMR (400 MHz, CDCl₃) δ 6.89 (dd, *J* = 15.8, 6.5 Hz, 1H), 5.70 (dd, *J* = 15. 8, 1.5 Hz, 1H), 5.19 (dd,*J* = 7.9, 2.9 Hz, 1H), 5.05 (d, *J* = 10.3 Hz, 1H), 4.18 (s, 2H), 2.62 (dd,*J* = 14.6, 7.8 Hz, 1H), 2.38 (brs, 1H), 2.15-2.06 (m, 3H), 1.98-1.90(m, 1H), 1.88-1.81 (m, 1H), 1.78-1.71 (m, 6H), 1.70-1.60 (m, 3H),1.53-1.45 (m, 2H), 1.31-1.22 (m, 4H), 1.19 (s, 3H), 0.99 (d, *J* = 6.8Hz, 3H), 0.93 (d, *J* = 7.0 Hz, 3H). 0.91 (d, *J*= 7.0 Hz, 3H). ¹³C NMR(100 MHz, CDCl₃) δ 173.0, 165.7, 155.0, 118.6, 85.5, 84.4, 76.5,70.6, 60.6, 47.4, 46.9, 40.4, 39.9, 33.0, 31.6 (2C), 31.1, 30.9, 25.9,25.7 (2C), 24.5, 18.9, 18.2, 17.4, 16.9. IR (KBr film) (cm⁻¹) ν 3438,2928, 2853, 1724, 1648. HPLC-ESI-HRMS ([M+H]⁺) calcd forC₂₆H₄₁O₆ 449.2898, found 449.2895. |
| 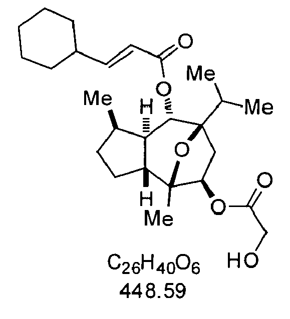 | |
| **16** | [α]_{D}²⁰ -28.5° (*c* 0.45, CHCl₃).¹H NMR (400 MHz, CDCl₃) δ 8.56 (s, 1H), 8.02 (dd, *J* = 8.5, 1.5 Hz, 1H), 7.96 (d, *J* = 7.8 Hz, 1H), 7.89 (d,*J =* 8.5 Hz, 2H), 7.60 (ddd, *J =* 8.0, 7.0, 1.2 Hz, 1H), 7.56 (ddd, *J =*8.3, 6.8, 1.5 Hz, 1H), 5.33 (d, *J* = 10.3 Hz, 1H), 5.28 (dd, *J* = 7.8, 3.0Hz, 1H), 4.22 (s, 2H), 2.86 (dd, *J* = 14.6, 7.8 Hz, 1H), 2.39 (brs, 1H),2.16 (sextet, *J* = 7.0 Hz, 1H), 1.98-1.89 (m, 1H), 1.94 (septet, *J* = 7.0Hz, 1H), 1.89 (dd, *J* = 14.6, 3.0 Hz, 1H), 1.89-1.65 (m, 3H), 1.27 (m,2H), 1.25 (s, 3H), 1.03 (d, *J* = 6.8 Hz, 3H), 0.99 (d, *J* = 7.3 Hz, 3H),0.97 (d, *J* = 7.3 Hz, 3H). ¹³C NMR (100 MHz, CDCl₃) δ 173.3, 165.4,135.7, 132.6, 131.3, 129.5, 128.5, 128.4, 127.9, 127.4, 126.9, 125.4,85.8, 84.7, 76.7, 71.8, 60.8, 47.7, 47.2, 40.4, 33.3, 31.3, 31.1, 24.8,19.2, 18.5, 17.6, 17.1. IR (KBr film) (cm⁻¹) ν 3468, 2962, 2879, 2364,1750, 1700. HPLC-ESI-HRMS ([M+H]⁺) calcd for C₃₀H₃₅O₆467.2428, found 467.2426. |
| 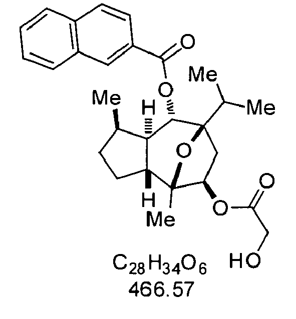 | |
| **17** | [α]_{D}²⁰ -47.8° (*c* 1.69, CHCl₃). ¹H NMR (400 MHz, CDCl₃) δ 7.49-7.46 (m, 2H), 7.39-7.36 (m, 3H), 6.07 (d, *J* = 1.0 Hz, 1H), 5.21 (dd,*J* = 7.9, 2.9 Hz, 1H), 5.10 (d, *J* = 10.0 Hz, 1H), 4.19 (s, 2H), 2.62 (dd,*J* = 14.6, 8.0 Hz, 1H), 2.58 (d, *J* = 0.8 Hz, 3H), 2.42 (brs, 1H), 2.16(sextet, *J =* 6.8 Hz, 1H), 2.00-1.94 (m, 1H), 1.90 (quintet, *J* = 7.0 Hz,1H), 1.79-1.72 (m, 3H), 1.52 (ddd, *J* = 13.1, 10.2, 6.5 Hz, 1H), 1.27-1.24 (m, 2H), 1.20 (s, 3H), 1.02 (d, *J* = 6.8 Hz, 3H), 0.98 (d, *J* = 7.3Hz, 3H), 0.96 (d, *J* = 7.3 Hz, 3H). ¹³C NMR (100 MHz, CDCl₃)δ 173.0, 165.3, 156.1, 142.1, 129.1, 128.5 (2C), 126.3 (2C), 117.0,85.6, 84.5, 76.4, 70.3, 60.6, 47.4, 47.0, 39.8, 32.9, 31.2, 30.9, 24.6,19.0, 18.2, 18.1, 17.5, 17.0. IR (KBr film) (cm⁻¹) ν 3462, 2959, 2875,1714, 1625. HPLC-ESI-HRMS ([M+H]⁺) calcd for C₂₇H₃₇O₆457.2585, found 457.2582. |
| 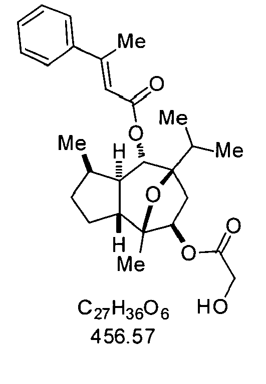 | |
| **18** | [α]_{D}²⁰ -53.0° (*c* 0.94, CHCl₃). ¹H NMR (400 MHz, CDCl₃) δ 7.62 (d, *J* = 15.8 Hz, 1H), 7.51 (dd, *J =* 8.7, 5.4 Hz, 2H), 7.07 (t, *J =* 8.5Hz, 2H), 6.31 (d, *J =* 15.8 Hz, 1H), 5.20 (dd, *J =* 7.8, 2.8 Hz, 1H),5.12 (d, *J* = 10.3 Hz, 1H), 4.19 (s, 2H), 2.66 (dd, *J* = 14.7, 7.9 Hz,1H), 2.57 (brs, 1H), 2.17-2.07 (m, 1H), 1.98-1.84 (m, 2H), 1.81-1.69 (m, 3H), 1.57-1.50 (m, 1H), 1.28-1.26 (m, 2H), 1.20 (s, 3H),1.00 (d, *J* = 7.0 Hz, 3H), 0.95 (d, *J* = 7.0 Hz, 3H), 0.93 (d, *J* = 7.0Hz, 3H). ¹³C NMR (100 MHz, CDCl₃) δ 173.0, 165.4, 143.9,130.4 (d, *J* = 2.9 Hz), 130.0 (d, *J* = 7.8 Hz, 2C), 117.6 (d, *J* = 2.9Hz), 116.0 (d, *J* = 21.4 Hz, 2C), 85.4, 84.5, 76.3, 71.1, 60.6, 60.4,47.4, 46.9, 39.9, 32.9, 31.1, 30.9, 24.5, 18.9, 18.2, 17.4, 16.9. IR(KBr film) (cm⁻¹) ν 3466, 2959, 1746, 1714. HPLC-ESI-HRMS([M+H]⁺) calcd for C₂₆H₃₄O₆F 461.2334, found 461.2330. |
| 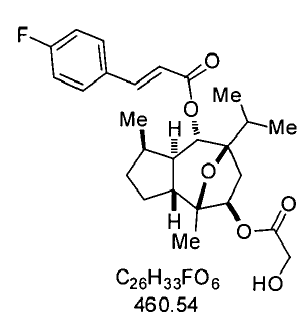 | |
| **19** | [α]_{D}²⁰ -47.6° (c 0.42, CHCl₃). ¹H NMR (400 MHz, CDCl₃) δ 7.61 (d, *J* = 16.1 Hz, 1H), 7.46 (d, *J* = 8.4 Hz, 2H), 7.3 6 (d, *J* = 8.4 Hz,2H), 6.36 (d, *J* = 16.1 Hz, 1H), 5.21 (dd, *J* = 7.8, 2.8 Hz, 1H), 5.13(d, *J* = 10.3 Hz, 1H), 4.20 (d, *J =* 5.3 Hz, 2H), 2.67 (dd, *J =* 14.6,7.8 Hz, 1H), 2.35 (t, *J =* 5.3 Hz, 1H), 2.14 (sextet, *J* = 6.8 Hz, 1H),1.95 (m, 1H), 1.88 (septet, *J* = 7.0 Hz, 1H), 1.81-1.69 (m, 2H),1.80 (dd, *J* = 14.6, 2.8 Hz, 1H), 1.55 (m, 1H), 1.26 (m, 2H), 1.21(s, 3H), 1.01 (d, *J* = 6.8 Hz, 3H), 0.96 (d, *J* = 7.0 Hz, 3H), 0.94 (d,*J* = 7.0 Hz, 3H). ¹³C NMR (100 MHz, CDCl₃) δ 173.2, 165.5,143.9, 136.5, 132.9, 129.4 (2C), 129.3 (2C), 118.6, 85.6, 84.7,76.6, 71.4, 60.8, 47.6, 47.1, 40.1, 33.1, 31.3, 31.1, 24.7, 19.1, 18.4,17.6, 17.0. IR (KBr film) (cm⁻¹) ν 3465, 2959, 2874, 1713. HPLC-ESI-HRMS ([M+H]⁺) calcd for C₂₆H₃₄O₆Cl 477.2038, found477.2035; ([M+H]⁺) calcd for C₂₆H₃₄O₆³⁷Cl 479.2009, found479.2005. |
| 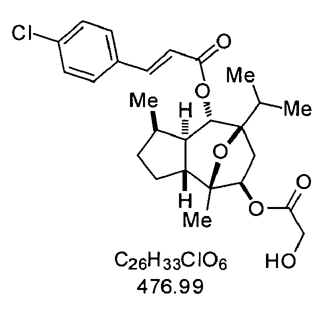 | |
| **20** | [α]_{D}²⁰ -44.4° (*c* 0.94, CHCl₃). ¹H NMR (400 MHz, CDCl₃) δ 7.59 (d, *J* = 15.8 Hz, 1H), 7.52 (d, *J* = 8.5 Hz, 2H), 7.83 (d, *J* = 8.5 Hz,2H), 6.37 (d, *J* = 16.1 Hz, 1H), 5.21 (dd, *J* = 7.9, 2.9 Hz, 1H), 5.13(d, *J* = 10.3 Hz, 1H), 4.19 (s, 2H), 2.66 (dd, *J* = 14.6, 8.0 Hz, 1H),2.42 (brs, 1H), 2.18-2.09 (m, 2H), 1.99-1.84 (m, 3H), 1.82-1.69(m, 3H), 1.57-1.50 (m, 1H), 1.20 (s, 3H), 1.01 (d, *J* = 6.8 Hz, 3H),0.96 (d, *J* = 7.0 Hz, 3H), 0.93 (d, *J* = 7.03 Hz, 3H). ¹³C NMR(100 MHz, CDCl₃) δ 173.0, 165.3, 143.8, 133.1, 132.1 (2C), 129.5(2C), 124.7, 118.6, 85.4, 84.5, 76.4, 71.2, 60.6, 47.4, 46.9, 39.9,32.9, 31.1, 30.9, 24.6, 18.9, 18.2, 17.4, 16.9. IR (KBr film) (cm⁻¹)ν 3450, 2958, 1711. HPLC-ESI-HRMS ([M+H]⁺) calcd forC₂₆H₃₄O₆Br 521.1533, found 521.1528; ([M+H]⁺) calcd forC₂₆H₃₄O₆⁸¹Br 523.1513, found 523.1508. |
| 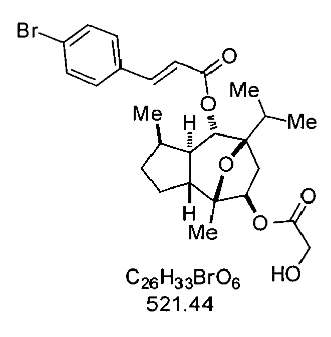 | |
| **21** | [α]_{D}²⁰ -47.7° (*c* 1.46, CHCl₃). ¹H NMR (400 MHz, CDCl₃) δ 8.25 (d, *J* = 8.8 Hz, 2H), 7.71-7.64 (m, 3H), 6.51 (d, *J* = 16.1 Hz, 1H),5.21 (dd, *J* = 7.8, 2.8 Hz, 1H), 5.15 (d, *J* = 10.3 Hz, 1H), 4.20 (m,2H), 2.66 (dd, *J* = 14.6, 8.0 Hz, 1H), 2.40 (brs, 1H), 2.16-2.09 (m,1H), 2.00-1.92 (m, 1H), 1.90-1.85 (m, 1H), 1.83-1.70 (m, 3H),1.66-1.52 (m, 3H), 1.21 (s, 3H), 1.01 (d, *J*= 6.8 Hz, 3H), 0.96 (d,*J* = 7.0 Hz, 3H), 0.94 (d, *J* = 7.3 Hz, 3H). ¹³C NMR (100 MHz,CDCl₃) δ 173.1, 164.7, 148.5, 142.2, 140.3, 128.7 (2C), 124.2(2C), 122.2, 85.4, 84.5, 76.3, 71.7, 60.6, 47.5, 46.9, 39.9, 32.9,31.2, 30.9, 24.6, 18.9, 18.2, 17.4, 16.9. IR (KBr film) (cm⁻¹) ν3447, 2963, 1714. HPLC-ESI-HRMS ([M+H]⁺) calcd forC₂₆H₃₄O₈N 488.2279, found 488.2276; ([M+NH₄]⁺) calcd for C₂₆H₃₇O₈N₂ 505.2544, found 505.2541; ([M+Na]⁺) calcd for C₂₆H₃₃O₈NNa 510.2098, found 510.2087. |
| 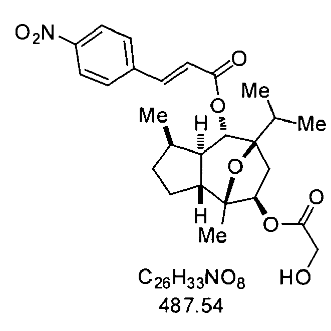 | |
| **22** | [α]_{D}²⁰ -46.4° (*c* 0.37, CHCl₃). ¹H NMR (200 MHz, CDCl₃) δ 7.64 (d, *J* = 16.1 Hz, 1H), 7.46 (d, *J* = 8.3 Hz, 2H), 7.26-7.22 (m, 3H), 6.34 (d, *J =* 16.1 Hz, 1H), 5.22 (dd, *J* = 7.8, 2.9 Hz, 1H), 5.13 (d, *J* = 10.3 Hz, 1H), 4.19 (brs, 2H), 3.00-2.85 (m, 1H), 2.69 (dd, *J* = 14.7, 7.8 Hz, 1H), 2.36 (brs, 1H), 2.21-2.07 (m, 1H), 1.98-1.82 (m, 3H), 1.78-1.70 (m, 3H), 1.61-1.54 (m, 4H), 1.24-1.21 (m, 6H), 1.01 (d, *J* = 6.8 Hz, 3H), 0.97 (d, *J* = 3.4 Hz, 3H), 0.93 (d, *J* = 3.4 Hz, 3H). ¹³C NMR (100 MHz, CDCl₃) δ 173.1, 165.8, 151.8, 145.2, 131.9, 129.7, 128.2 (2C), 127.0 (2C), 116.9, 85.5, 84.5, 76.5, 71.0, 60.6, 47.4, 46.9, 39.9, 34.1, 33.1, 31.2, 30.9, 24.6, 23.8, 19.0, 18.2, 17.5, 16.9. IR (KBr film) (cm⁻¹) ν 3388, 2959, 2927, 1713. HPLC-ESI-HRMS ([M+H]⁺) calcd for C₂₉H₄₁O₆ 485.2898, found 485.2892. |
| 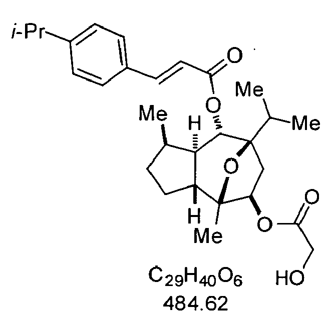 | |
| **23** | [α]_{D}²⁰ -46.3° (*c* 1.20, CHCl₃). ¹H NMR (400 MHz, CDCl₃) δ 7.56 (d, *J* = 16.1 Hz, 1H), 7.03-6.98 (m, 2H), 6.83-6.77 (m, 1H), 6.20 (d, *J* = 15.8 Hz, 1H), 6.00-5.99 (m, 2H), 5.21 (dd, *J* = 7.9, 2.9 Hz, 1H), 5.11 (d, *J =* 10.3 Hz, 1H), 4.19-4.17 (m, 2H), 2.66 (dd, *J =* 14.6, 8.0 Hz, 1H), 2.49-2.47 (m, 1H), 2.17-2.09 (m, 1H), 1.98-1.83 (m, 2H), 1.81-1.67 (m, 3H), 1.57-1.49 (m, 1H), 1.27-1.23 (m, 2H), 1.20 (s, 3H), 1.00 (d, *J* = 6.8 Hz, 3H), 0.97-0.94 (m, 3H), 0.93 (d, *J* = 7.3 Hz, 3H). ¹³C NMR (100 MHz, CDCl₃) δ 173.0, 165.7, 149.7, 148.3, 144.9, 128.6, 124.6, 115.8, 108.5, 106.4, 101.6, 85.5, 84.5, 76.4, 70.9, 60.6, 47.4, 46.9, 39.9, 33.0, 31.1, 30.9, 24.6, 18.9, 18.2, 17.4, 16.9. IR (KBr film) (cm⁻¹) ν 3424, 2959, 1710. HPLC-ESI-HRMS ([M+H]⁺) calcd for C₂₇H₃₅O₈ 487.2326, found 487.2320. |
| 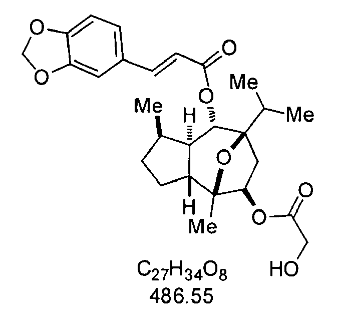 | |
| **24** | [α]_{D}²⁰ -56.1° (*c* 1.22, CHCl₃). ¹H NMR (400 MHz, CDCl₃) δ 7.90 (d, *J* = 16.3 Hz, 1H), 7.29-7.25 (m, 2H), 7.08-7.02 (m, 1H), 6.67 (d, *J* = 16.6 Hz, 1H), 5.23 (dd, *J* = 7.8, 2.8 Hz, 1H), 5.14 (d, *J* = 10.3 Hz, 1H), 4.19 (s, 2H), 2.67 (dd, *J* = 14.6, 7.8 Hz, 1H), 2.41 (brs, 1H), 2.16 (sextet, *J* = 7.1 Hz, 1H), 1.99-1.86 (m, 2H), 1.83-1.70 (m, 3H), 1.60-1.52 (m, 1H), 1.31-1.24 (m, 2H), 1.20 (s, 3H), 1.02 (d, *J* = 7.0 Hz, 3H), 0.97 (d, *J* = 7.3 Hz, 3H), 0.95 (d, *J* = 7.3 Hz, 3H). ¹³C NMR (100 MHz, CDCl₃) δ 173.0, 165.4, 136.1 (d, *J* = 5.8 Hz), 135.2 (d, *J* = 1.9 Hz), 130.9, 130.8, 126.0 (d, *J =* 2.9 Hz), 124.9, 124.8, 115.0, 85.4, 84.5, 76.4, 71.4, 60.6, 47.4, 46.9, 39.8, 35.1, 31.1, 30.9, 24.5, 18.9, 18.2, 17.5, 16.9. IR (KBr film) (cm⁻¹) ν 3459, 2960, 1717. HPLC-ESI-HRMS ([M+H]⁺) calcd for C₂₆H₃₃O₆ClF 495.1944, found 495.1939; ([M+H]⁺) calcd for C₂₆H₃₃O6³⁷ClF 497.1915, found 497.1910. |
| 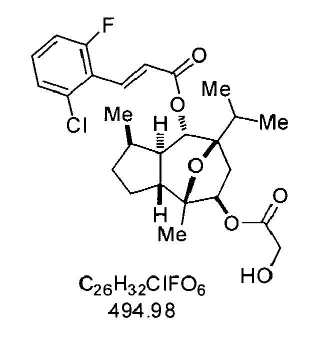 | |
| **25** | [α]_{D}²⁰ -42.2° (*c* 1_{.}04, CHCl₃)_{.} ¹H NMR (400 MHz, CDCl₃) δ 7.56 (d, *J* = 15.8 Hz, 1H), 6.74 (s, 2H), 6.28 (d, *J* = 15.8 Hz, 1H), 5.19 (dd, *J* = 7.8, 2.8 Hz, 1H), 5.13 (d, *J* = 10.3 Hz, 1H), 4.20-4.18 (m, 2H), 3.89-3.87 (m, 9H), 2.70 (dd, *J* = 14.6, 7.8 Hz, 1H), 2.42 (brs, 1H), 2.13 (septet, *J* = 6.7 Hz, 1H), 1.97-1.85 (m, 2H), 1.81-1.70 (m, 3H), 1.54 (ddd, *J* = 13.2, 10.3, 6.4 Hz, 1H), 1.27-1.23 (m, 2H), 1.21 (s, 3H), 1.01 (d, *J* = 6.8 Hz, 3H), 0.96 (d, *J* = 7.0 Hz, 3H), 0.93 (d, *J* = 7.0 Hz, 3H). ¹³C NMR (100 MHz, CDCl₃) δ 173.0, 165.5, 153.4 (2C), 145.1, 140.1, 129.7, 117.2, 105.2 (2C), 85.5, 84.4, 76.4, 71.0, 61.0, 60.6, 56.1 (2C), 47.4, 46.9, 40.0, 33.0, 31.1, 30.9, 24.6, 19.0, 18.2, 17.4, 16.9. IR (KBr film) (cm⁻¹) ν 3458, 2959, 1709. HPLC-ESI- HRMS ([M+H]⁺) calcd for C₂₆H₄₁O₉ 533.2745, found 533.2740; ([M+NH₄]⁺) calcd for C₂₆H₄₄O₉N 550.3011, found 550.3006; ([M+Na]⁺) calcd for C₂₆H₄₀O₉Na 555.2565, found 555.2556. |
| 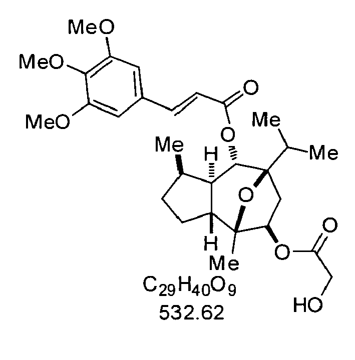 | |
| **26** | [α]_{D}²⁰ -52.5° (*c* 1.17, CHCl₃). ¹H NMR (400 MHz, CDCl₃) δ 7.64 (d, *J* = 1.3 Hz, 1H), 7.40-7.30 (m, 4H), 7.33 (sextet, *J* = 8.7 Hz, 1H), 5.22 (dd, *J =* 7.9, 2.9 Hz, 1H), 5.15 (d, *J =* 10.0 Hz, 1H), 4.20 (s, 2H), 2.69 (dd, *J* = 14.4, 7.9 Hz, 1H), 2.40 (brs, 1H), 2.16 (sextet, *J* = 6.8 Hz, 1H), 2.10 (d, J = 1.25 Hz, 3H), 1.99-1.93 (m,1H), 1.89 (quintet, *J* = 6.8 Hz, 1H), 1.83-1.73 (m, 3H), 1.61-1.55 (m, 1H), 1.31-1.24 (m, 2H), 1.22 (s, 3H), 1.02 (d, *J* = 6.8 Hz, 3H), 0.97 (d, *J* = 7.8 Hz, 3H), 0.95 (d, *J* = 7.8 Hz, 3H). ¹³C NMR (100 MHz, CDCl₃) δ 173.1, 167.1, 139.3, 135.7, 129.7 (2C), 128.4 (2C), 128.2, 85.6, 84.5, 76.5, 71.3, 60.6, 47.4, 47.0, 40.0, 33.0, 31.2, 30.9, 26.9, 24.6, 19.0, 18.2, 17.4, 16.9, 14.2. IR (KBr film) (cm⁻¹) ν 3469, 2960, 2875, 1745, 1707. HPLC-ESI-HRMS ([M+H]⁺) calcd for C₂₇H₃₇O₆ 457.2585, found 457.2583. |
| 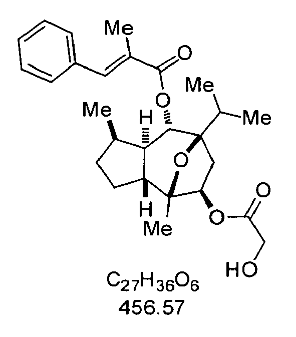 | |
| **27** | [α]_{D}²⁰ -44.6° (*c* 0.38, CHCl₃). ¹H NMR (300 MHz, CDCl₃) δ 8.01 (ddd, *J* = 7.3, 1.8, 1.5 Hz, 2H), 7.57 (tdd, *J* = 7.3, 1.8, 1.5 Hz, 1H), 7.45 (tt, *J =* 7.3, 1.5 Hz, 2H), 5.26 (d, *J* = 9.9 Hz, 1H), 5.24 (dd, *J* = 7.7, 2.9 Hz, 1H), 4.21 (d, *J* = 5.5 Hz, 2H), 2.78 (dd, *J* = 14.6, 7.7 Hz, 1H), 2.36 (t, *J =* 5.5 Hz, 1H), 2.13 (sextet, *J =* 7.0 Hz, 1H), 1.97-1.72 (m, 5H), 1.63 (m, 1H), 1.26 (m, 2H), 1.23 (s, 3H), 1.01 (d, *J =* 7.0 Hz, 3H), 0.96 (d, *J* = 7.0 Hz, 3H), 0.95 (d, *J* = 7.0 Hz, 3H). ¹³C NMR (125 MHz, CDCl₃) δ 173.2, 165.2, 133.2, 130.3, 129.8 (2C), 128.6 (2C), 85.7, 84.7, 76.7, 71.7, 60.8, 47.7, 47.2, 40.2, 33.1, 31.4, 31.1, 24.8, 19.1, 18.4, 17.6, 17.1. IR (KBr film) (cm⁻¹) ν 3468, 2958, 2874, 1720. HPLC-ESI-HRMS ([M+H]⁺) calcd for C₂₄H₃₃O₆ 417.2272, found 417.2271. |
| 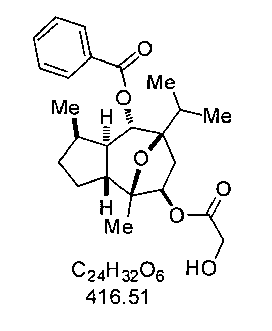 | |
| **28** | [α]_{D}²⁰ -36.0° (*c* 0.67, CHCl₃). ¹H NMR (400 MHz, CDCl₃) δ 8.02 (ddd, *J* = 8.8, 5.3, 2.0 Hz, 2H), 7.12 (ddd, *J =* 8.8, 8.5, 1.8 Hz, 2H), 5.24 (d, *J =* 10.3 Hz, 1H), 5.23 (dd, *J*= 7.5, 3.3 Hz, 1H), 4.21 (s, 2H), 2.74 (dd, *J* = 14.6, 7.8 Hz, 1H), 2.36 (brs, 1H), 2.12 (sextet, *J* = 6.8 Hz, 1H), 2.00-1.71 (m, 5H), 1.60 (m, 1H), 1.26 (m, 2H), 1.23 (s, 3H), 1.01 (d, *J* = 6.8 Hz, 3H), 0.96 (d, *J* = 6.8 Hz, 3H), 0.94 (d, *J* = 6.8 Hz, 3H). ¹³C NMR (100 MHz, CDCl₃) δ 173.2, 164.7, 164.2, 132.3 (d, *J* = 8.7 Hz, 2C), 126.4 (d, *J* = 3.9 Hz), 115.8 (d, *J* = 21.4 Hz, 2C), 85.6, 84.7, 76.6, 71.8, 60.8, 47.6, 47.1, 40.3, 33.2, 31.3, 31.1, 24.7, 19.1, 18.4, 17.6, 17.1. IR (KBr film) (cm⁻¹) ν 3471, 2960, 2876, 1722. HPLC-ESI-HRMS ([M+H]⁺) calcd for C₂₄H₃₂O₆F 435.2177, found 435.2176. |
| 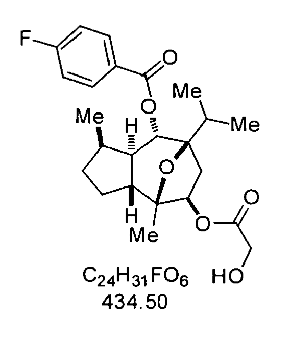 | |
| **29** | [α]_{D}²⁰ -35.0° (*c* 1.23, CHCl₃). ¹H NMR (400 MHz, CDCl₃) δ 7.93 (d, *J* = 8.5 Hz, 2H), 7.42 (d, *J* = 8.5 Hz, 2H), 5.26-5.22 (m, 2H), 4.20 (d, *J =* 4.0 Hz, 2H), 2.74 (dd, *J =* 14.6, 7.8 Hz, 1H), 2.38 (t, *J* = 4.8 Hz, 1H), 2.12 (quintet, *J* = 7.3 Hz, 1H), 1.96-1.71 (m, 5H), 1.65-1.57 (m, 2H), 1.27-1.24 (m, 1H), 1.22 (s, 3H), 1.00 (d, *J* = 7.0 Hz, 3H), 0.96-0.91 (m, 6H). ¹³C NMR (100 MHz, CDCl₃) δ 173.1, 164.2, 139.6, 131.0 (2C), 128.8 (2C), 128.5, 85.5, 84.6, 76.4, 71.8, 60.6, 47.5, 46.9, 40.1, 33.1, 31.1, 30.9, 24.6, 19.0, 18.3, 17.4, 16.9. IR (KBr film) (cm⁻¹) ν 3471, 2960, 2876, 1722. HPLC-ESI-HRMS ([M+H]⁺)calcd for C₂₄H₃₂O₆³⁷Cl 453.1852, found 453.1851; calcd for C₂₄H_{3 2}O₆Cl 451.1882, found 451.1880. |
| 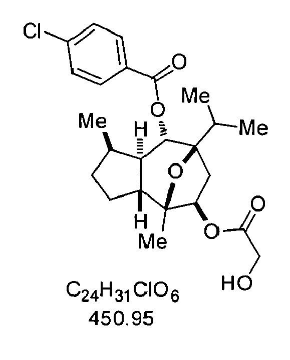 | |
| **30** | [α]_{D}²⁰ -26.7° (*c* 0.82, CHCl₃). ¹H NMR (400 MHz, CDCl₃) δ 8.30 (d, *J* = 8.8 Hz, 2H), 8.17 (d, *J* = 8.8 Hz, 2H), 5.29 (d, *J* = 10.0 Hz, 1H), 5.24 (dd, *J =* 7.8, 2.8 Hz, 1H), 4.21 (s, 2H), 2.75 (dd, *J =* 14.6, 7.8 Hz, 1H), 2.38 (brs, 1H), 2.14-2.09 (m, 1H), 1.98-1.91 (m, 1H), 1.89-1.84 (m, 2H), 1.82-1.73 (m, 2H), 1.68-1.61 (m, 3H), 1.23 (s, 3H), 1.01 (d, *J* = 6.8 Hz, 3H), 0.96 (d, *J* = 7.3 Hz, 3H), 0.94 (d, *J* = 7.3 Hz, 3H). ¹³C NMR (100 MHz, CDCl₃) δ 173.1, 163.2, 150.6, 135.4, 130.8 (2C), 123.6 (2C), 85.3, 84.6, 76.3, 72.7, 60.6, 47.5, 46.9, 40.1, 33.0, 31.1, 30.9, 24.6, 18.9, 18.3, 17.4, 16.9. IR (KBr film) (cm⁻¹) ν 3468, 2959, 2875, 1725. HPLC-ESI-HRMS ([M+H]⁺) calcd for C₂₄H₃₂O₈N 462.2122, found 462.2121. |
| 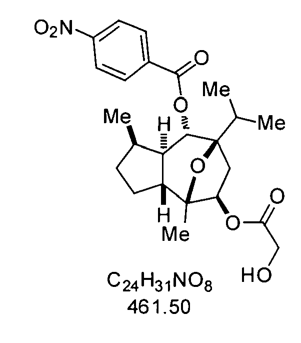 | |
| **31** | [α]_{D}²⁰ -40.4° (*c* 0.75, CHCl₃). ¹H NMR (400 MHz, CDCl₃) δ 8.06 (d, *J* = 1.8 Hz, 1H), 7.82 (dd, *J* = 8.3, 1.8 Hz, 1H), 7.53 (d, *J* = 8.3 Hz, 1H), 5.25 (d, *J* = 10.5 Hz, 1H), 5.24 (d, *J* = 7.3, 3.0 Hz, 1H), 4.21 (s, 2H), 2.73 (dd, *J* = 14.6, 8.0 Hz, 1H), 2.35 (dd, *J* = 7.8, 7.3 Hz, 1H), 2.10 (sextet, *J* = 6.8 Hz, 1H), 1.95 (m, 1H), 1.92-1.71 (m, 4H), 1.63 (m, 1H), 1.26 (m, 2H), 1.23 (s, 3H), 1.00 (d, *J* = 6.8 Hz, 3H), 0.94 (d, *J* = 7.0 Hz, 3H), 0.93 (d, *J* = 7.0 Hz, 3H). ¹³C NMR (100 MHz, CDCl₃) δ 173.3, 163.4, 138.0, 133.2, 131.7, 130.8, 130.0, 128.9, 85.6, 84.7, 76.5, 72.4, 60.8, 47.7, 47.0, 40.3, 33.2, 31.3, 31.0, 24.8, 19.1, 18.4, 17.5, 17.0. IR (KBr film) (cm⁻¹) ν 3435, 2958, 2356, 1724. HPLC-ESI-HRMS ([M+H]⁺) calcd for C₂₄H₃₁O₆Cl₂ 485.1492, found 485.1488; ([M+H]⁺) calcd for C₂₄H₃₁O₆Cl³⁷Cl 487.1463, found 487.1458; ([M+H]⁺) calcd for C₂₄H₃₁O₆³⁷Cl₂ 489.1433, found 489.1426. |
| 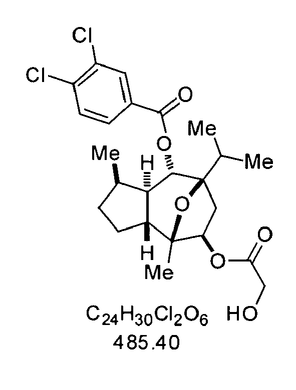 | |
| **32** | [α]_{D}²⁰ -36.9° (*c* 0.91, CHCl₃). ¹H NMR (400 MHz, CDCl₃) δ 8.78-8.77 (m, 1H), 8.05 (d, *J* = 7.8 Hz, 1H), 7.83 (td, *J* = 7.8, 1.8 Hz,1H), 7.47 (ddd, *J* = 7.5, 4.8, 1.3 Hz, 1H), 5.33 (d, *J =* 10.3 Hz,1H), 5.24 (dd, *J =* 7.9, 2.9 Hz, 1H), 4.20 (s, 2H), 2.81 (dd, *J =* 14.6, 8.0 Hz, 1H), 2.53 (brs, 1H), 2.18-2.10 (m, 1H), 1.95-1.89 (m,2H), 1.87-1.78 (m, 3H), 1.76-1.65 (m, 2H), 1.27-1.26 (m, 1H), 1.21 (s, 3H), 1.01 (d, *J* = 6.8 Hz, 3H), 0.97 (d, *J* = 2.8 Hz, 3H),0.95 (d, *J* = 2.8 Hz, 3H). ¹³C NMR (100 MHz, CDCl₃) δ 173.0,163.6, 150.1, 147.9, 137.0, 126.9, 125.1, 85.5, 84.6, 76.4, 72.5, 60.6, 47.5, 46.8, 39.9, 32.8, 31.1, 30.9, 24.6, 18.9, 18.2, 17.4, 16.9. IR (KBr film) (cm⁻¹) ν 3461, 2959, 2876, 1739. HPLC-ESI-HRMS ([M+Na]⁺) calcd for C₂₃H₃₁O₆NNa 440.2044, found 440.2038. |
| 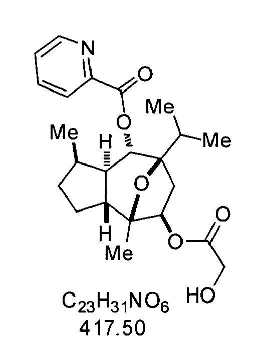 | |
| **33** | [α]_{D}²⁰ -16.9° (*c* 0.54, CHCl₃). ¹H NMR (400 MHz, CDCl₃) δ 5.18 (dd, *J* = 7.9, 2.9 Hz, 1H), 4.99 (d, *J* = 10.3 Hz, 1H), 4.18 (d, *J* = 5.02, 2H), 2.58 (dd, *J =* 14.6, 8.0 Hz, 1H), 2.35 (t, *J =* 5.4 Hz, 1H), 2.13-2.08 (m, 1H), 2.02 (s, 3H), 1.96-1.91 (m, 1H), 1.86-1.81 (m, 1H), 1.77-1.68 (m, 3H), 1.58 (brs, 2H), 1.49-1.44 (m, 1H), 1.18 (s, 3H), 1.00 (d, *J* = 6.8 Hz, 3H), 0.94 (d, *J* = 7.0 Hz, 3H), 0.90 (d, *J* = 7.3 Hz, 3H). ¹³C NMR (100 MHz, CDCl₃) δ 173.0, 169.6, 85.3, 84.5, 76.4, 71.0, 60.6, 47.4, 46.8, 39.7, 32.9, 31.2, 30.9, 24.5, 21.3, 18.9, 18.1, 17.4, 16.8. IR (KBr film) (cm⁻¹) ν 3453, 2958, 2929, 1737. HPLC-ESI-HRMS ([M+H]⁺) calcd for C₁₉H₃₀O₆ 355.2115, found 355.2116. |
| 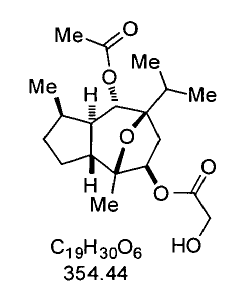 | |
| **34** | [α]_{D}²⁰ -33.9° (*c* 0.56, CHCl₃). ¹H NMR (500 MHz, CDCl₃) δ 6.95 (dq, *J =* 15.4, 6.9 Hz, 1H), 5.80 (dq, *J =* 15.4, 1.7 Hz, 1H), 5.19 (dd, *J* = 7.9, 3.0 Hz, 1H), 5.05 (d, *J* = 10.2 Hz, 1H), 4.18 (s, 2H), 2.61 (dd, *J =* 14.4, 7.9 Hz, 1H), 2.35 (brs, 1H), 2.09 (septet, *J =* 7.0 Hz, 1H), 1.94 (m, 1H), 1.88 (dd, *J* =6.9, 1.7 Hz, 3H), 1.85 (m, 1H), 1.76 (dd, *J =* 14.4, 3.0 Hz, 1H), 1.72 (m, 2H), 1.48 (m, 1H), 1.25 (m, 2H), 1.19 (s, 3H), 0.99 (d, *J* = 6.9 Hz, 3H), 0.93 (d, *J* = 7.0 Hz, 3H), 0.91 (d, *J* = 7.0 Hz, 3H). ¹³C NMR (125 MHz, CDCl₃) δ 173.2, 165.2, 145.3, 122.8, 85.6, 84.6, 76.6, 70.8, 60.8, 47.6, 47.1, 40.0, 33.1, 31.3, 31.1, 24.7, 19.1, 18.3, 18.1, 17.6, 17.0. IR (KBr film) (cm⁻¹) ν 3464, 2959, 1720. HPLC-ESI-HRMS ([M+H]⁺) calcd for C₂₁H₃₃O₆ 381.2272, found 381.2272; ([M+NH₄]⁺) calcd for C₂₁H₃₆O₆N 398.2537, found 398.2538; ([M+Na]⁺) calcd for C₂₁H₃₂O₆Na 403.2091, found 403.2091. |
| 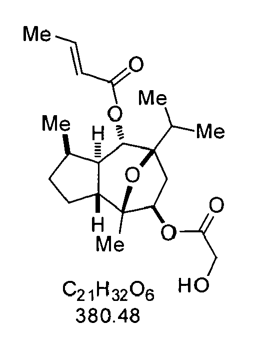 | |
| **35** | [α]_{D}²⁰ -31.6° (*c* 0.41, CHCl₃). ¹H NMR (500 MHz, CDCl₃) δ 6.81 (qd, *J =* 6.9, 1.5 Hz, 1H), 5.19 (dd, *J =* 7.7, 3.1 Hz, 1H), 5.07 (d, *J* = 9.9 Hz, 1H), 4.18 (d, *J* = 3.4 Hz, 1H), 3.64 (s, 1H), 2.63 (dd, *J* = 14.5, 8.0 Hz, 1H), 2.33 (m, 1H), 2.09 (sextet, *J* = 6.5 Hz, 1H), 1.94 (m, 1H), 1.84 (septet, *J* = 6.9 Hz, 1H), 1.81 (m, 3H), 1.79 (dd, *J* = 6.9, 1.5 Hz, 3H), 1.79-1.68 (m, 3H), 1.50 (m, 1H), 1.24 (m, 2H), 1.20 (s, 3H), 0.99 (d, *J=* 6.5 Hz, 3H), 0.93 (d, *J* = 6.9 Hz, 3H), 0.91 (d, *J* = 6.9 Hz, 3H). ¹³C NMR (100 MHz, CDCl₃) δ 173.2, 166.6, 137.7, 128.7, 85.7, 84.6, 76.7, 70.9, 60.8, 47.5, 47.2, 40.1, 33.1, 31.3, 31.1, 24.7, 19.1, 18.3, 17.5, 17.1, 14.6, 12.3. IR (KBr film) (cm⁻¹) ν 3470, 2957, 1744, 1709. HPLC-ESI-HRMS ([M+H]⁺) calcd for C₂₂H₃₅O₆ 395.2428, found 395.2428 |
| 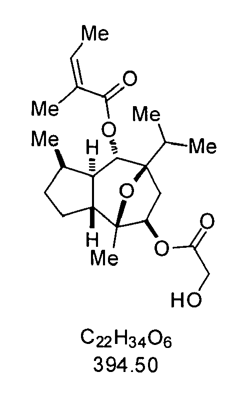 | |

### ANALYSIS OF THE COMPOUNDS 36-44

| | |
|---|---|
| **36** | [α]_{D}²⁰ -51.8° (*c* 0.94, CHCl₃). ¹H NMR (400 MHz, CDCl₃) δ 7.66 (d, *J* = 16.1 Hz, 1H), 7.52 (dd, *J* = 6.4, 2.9 Hz, 2H), 7.39-7.38 (m, 3H), 6.39 (d, *J* = 16.1 Hz, 1H), 5.20 (dd, *J* = 7.8, 2.8 Hz, 1H), 5.13 (d, *J* = 10.3 Hz, 1H), 4.06 (s, 2H), 3.47 (s, 3H), 2.67 (dd, *J* = 14.6, 7.8 Hz, 1H), 2.18-2.09 (m, 1H), 1.99-1.86 (m, 2H), 1.81-1.70 (m, 3H), 1.60-1.52 (m, 1H), 1.29-1.25 (m, 2H), 1.22 (s, 3H), 1.01 (d, *J* = 6.8 Hz, 3H), 0.96 (d, *J* = 7.0 Hz, 3H), 0.94 (d, *J* = 7.0 Hz, 3H). ¹³C NMR (100 MHz, CDCl₃) δ 170.0, 165.6, 145.1, 134.2, 130.3, 128.9 (2C), 128.1 (2C), 117.9, 85.4, 84.5, 75.5, 71.1, 69.7, 59.4, 47.5, 46.9, 40.0, 33.0, 31.1, 30.9, 24.5, 19.0, 18.2, 17.4, 16.9. IR (KBr film) (cm⁻¹) ν 1710, 1635. HPLC-ESI-HRMS ([M+H]⁺) calcd for C₂₇H₃₇O₆ 457.2585, found 457.2581; ([M+Na]⁺) calcd for C₂₇H₃₆O₆Na 479.2404, found 479.2400. |
| 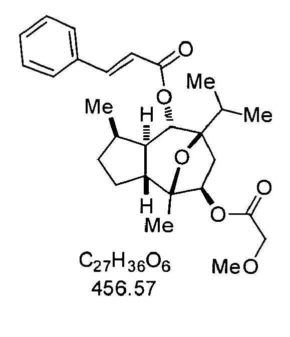 | |
| **37** | [α]_{D}²⁰ -47.7° (*c* 0.64, CHCl₃). ¹H NMR (400 MHz, CDCl₃) δ 7.66 (d, *J* = 16.1 Hz, 1H), 7.54-7.51 (m, 2H), 7.40-7.38 (m, 3H), 6.39 (d, *J* = 15.8 Hz, 1H), 5.17 (dd, *J* = 7.8, 2.8 Hz, 1H), 5.12 (d, *J* = 10.3 Hz, 1H), 4.65 (s, 2H), 2.65 (dd, *J* = 14.4, 7.9 Hz, 1H), 2.59 (t, *J* = 7.4 Hz, 2H), 2.31 (td, *J* = 6.9, 2.5 Hz, 2H), 2.13 (q, *J* = 13.8, 6.8 Hz, 1H), 1.98 (t, *J* = 2.6 Hz, 1H), 1.90 (dt, *J* = 14.0, 6.9 Hz, 2H), 1.82-1.68 (m, 3H), 1.63-1.49 (m, 3H), 1.27-1.24 (m, 2H), 1.21 (s, 3H), 1.01 (d, *J* = 6.8 Hz, 3H), 0.97 (d, *J* = 7.0 Hz, 3H), 0.94 (d, *J* = 7.3 Hz, 3H). ¹³C NMR (101 MHz, CDCl₃) δ 172.4, 167.6, 165.6, 145.2, 134.2, 130.4, 128.9 (2C), 128.1 (2C), 117.9, 85.5, 84.5, 83.1, 76.3, 71.1, 69.3, 60.7, 47.5, 46.9, 39.8, 32.9, 32.4, 31.2, 30.9, 24.6, 23.5, 19.0, 18.2, 17.8, 17.4, 16.9. IR (KBr film) (cm⁻¹) ν 3445, 2922, 1746, 1711, 1638. HPLC-ESI-HRMS ([M+H]⁺) calcd for C₃₂H₄₁O₇ 537.2847, found 537.2842; ([M+NH₄]⁺) calcd for C₃₂H₄₄O₇N 554.3143, found 554.3109. |
| 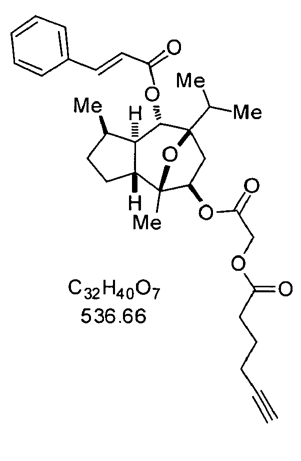 | |
| **38** | [α]_{D}²⁰ -45.4° (c 1.81, CHCl₃). ¹H NMR (400 MHz, CDCl₃) δ 7.65 (d, *J =* 16.1 Hz, 1H), 7.52 (dd, *J =* 6.4, 2.9 Hz, 2H), 7.38 (m, 3H), 6.39 (d, *J* = 16.1 Hz, 1H), 5.14-5.09 (m, 2H), 2.65 (dd, *J* = 14.4, 7.9 Hz, 1H), 2.50 (t, J= 7.4 Hz, 2H), 2.28 (td, J= 6.9, 2.5 Hz, 2H), 2.18-2.08 (m, 1H), 1.98 (t, J = 2.5 Hz, 1H), 1.95-1.90 (m, 1H), 1.89-1.83 (m, 3H), 1.78-1.68 (m, 3H), 1.60-1.52 (m, 1H), 1.25-1.24 (m, 2H), 1.21 (s, 3H), 1.02 (d, *J =* 6.8 Hz, 3H), 0.97 (d, *J =* 7.3 Hz, 3H), 0.94 (d, *J* = 7.0 Hz, 3H). ¹³C NMR (100 MHz, CDCl₃) δ 172.7, 165.6, 145.1, 134.2, 130.3, 128.9 (2C), 128.1 (2C), 118.0, 85.4, 84.5, 83.1, 75.0, 71.2, 69.2, 47.5, 46.9, 40.1, 33.0, 33.0, 31.2, 30.9, 24.6, 23.5, 19.0, 18.2, 17.8, 17.5, 16.9. IR (KBr film) (cm⁻¹) ν 3406, 2958, 1712, 1641. HPLC-ESI-HRMS ([M+H]⁺) calcd for C₃₀H₃₉O₅ 479.2792, found 479.2786; ([M+NH₄]⁺) calcd for C₃₀H₄₂O₅N 496.3058, found 496.3053. |
| 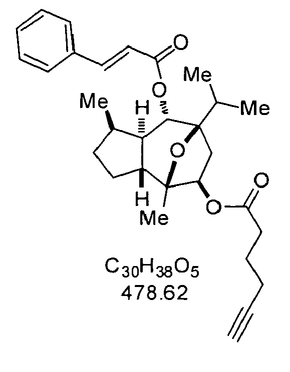 | |
| **39** | [α]_{D}²⁰ -41.7° (*c* 0.71, CHCl₃). ¹H NMR (400 MHz, CDCl₃) δ 7.66 (d, *J* = 16.1 Hz, 1H), 7.53 (m, 2H), 7.39 (m, 3H), 6.39 (d, *J* = 16.1 Hz, 1H), 5.19 (dd, *J* = 8.0, 3.0 Hz, 1H), 5.13 (d, *J* = 10.0 Hz, 1H), 4.34 (d, *J* = 2.5 Hz, 2H), 4.24 (s, 2H), 2.67 (dd, *J* = 14.6, 8.0 Hz, 1H), 2.49 (t, *J =* 2.3 Hz, 1H), 2.14 (sextet, *J =* 6.8 Hz, 1H), 1.94 (m, 1H), 1.89 (septet, *J =* 7.0 Hz, 1H), 1.80-1.68 (m, 2H), 1.79 (dd, *J =* 14.6, 3.0 Hz, 1H), 1.56 (m, 1H), 1.26 (m, 2H), 1.22 (s, 3H), 1.02 (d, *J* = 6.8 Hz, 3H), 0.97 (d, *J* = 7.0 Hz, 3H), 0.94 (d, *J* = 7.0 Hz, 3H). ¹³C NMR (100 MHz, CDCl₃) δ 169.8, 165.7, 145.3, 134.4, 130.6, 129.0 (2C), 128.3 (2C), 118.1, 85.6, 84.7, 78.6, 75.9, 75.8, 71.3, 66.2, 58.4, 47.6, 47.1, 40.1, 33.2, 31.3, 31.1, 24.7, 19.2, 18.4, 17.6, 17.1. IR (KBr film) (cm⁻¹) ν 3465, 3301, 2959, 1751, 1710, 1637. HPLC-ESI-HRMS ([M+H]⁺) calcd for C₂₉H₃₇O₆ 481.2585, found 481.2580; ([M+NH₄]⁺) calcd for C₂₉H₄₀O₆N 498.2850, found 498.2846; ([M+Na]⁺) calcd for C₂₉H₃₆O₆Na 503.2404, found 503.2399. |
| 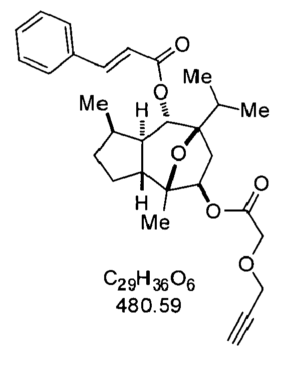 | |
| **40** | [α]_{D}²⁰ -42.5° (*c* 0.99, CHCl₃). ¹H NMR (300 MHz, CDCl₃, presence of rotamers) δ 7.66 (d, *J*= 16.1 Hz, 1H), 7.52 (m, 2H), 7.38 (m, 3H), 6.39 (d, *J* = 16.1 Hz, 1H), 5.13 (m, 2H), 4.01 (m, 2H), 2.95 & 2.93 (2s, 3H), 2.66 (m, 1H), 2.13 (sextet, *J* = 6.6 Hz, 1H), 1.93 (m, 1H), 1.88 (septet, *J* = 7.0 Hz, 1H), 1.78 (dd, *J* = 14.3, 2.2 Hz, 1H), 1.78-1.67 (m, 2H), 1.55 (m, 1H), 1.47 & 1.44 (s, 9H), 1.25 (m, 2H), 1.21 (s, 3H), 1.01 (d, *J* = 6.6 Hz, 3H), 0.96 (d, *J* = 7.0 Hz, 3H), 0.93 (d, *J* = 7.0 Hz, 3H). ¹³C NMR (125 MHz, CDCl₃, presence of rotamers) δ 169.8, 165.7, 156.1 & 155.8, 145.3 & 145.2, 134.4, 130.5, 129.0 (2C), 128.3 (2C), 118.2, 85.6, 84.6, 80.4 & 80.3, 76.0, 71.5 & 71.4, 51.3 & 50.7, 47.7, 47.1, 40.1, 35.8 & 35.7, 33.1 & 33.0, 31.3, 31.1, 28.5 (3C), 24.7, 19.2, 18.3, 17.6, 17.0. IR (KBr film) (cm⁻¹) v 3060, 2974, 2876, 1748, 1708, 1637. HPLC-ESI-HRMS ([M+H]⁺) calcd for C₃₂H₄₆O₇N 556.3269, found 556.3265; ([M+Na]⁺) calcd for C₃₂H₄₅O₇NNa 578.3088, found 578.3083. |
| 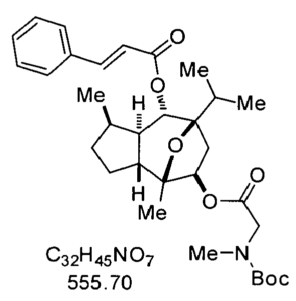 | |
| **41** | [α]_{D}²⁰ -40.5° (c 0.94, CHCl₃). ¹H NMR (500 MHz, CDCl₃) δ 7.66 (d, *J* = 16.1 Hz, 1H), 7.52 (m, 2H), 7.39 (m, 3H), 6.39 (d, *J* = 16.1 Hz, 1H), 5.18(dd, *J*=7.3, 2.9Hz, 1H), 5.13 (d, *J* = 11.0 Hz, 1H), 3.44 (d, *J* = 2.9 Hz, 2H), 2.67 (dd, *J* = 14.7, 8.1 Hz, 1H), 2.49 (s, 3H), 2.14 (sextet, *J* = 6.6 Hz, 1H), 2.11 (brs, 1H), 1.95 (m, 1H), 1.89 (septet, *J* = 7.3 Hz, 1H), 1.80-1.69 (m, 2H), 1.79 (dd, *J =* 14.7, 2.9Hz, 1H), 1.56 (m, 1H), 1.26 (m, 2H), 1.22 (s, 3H), 1.02 (d, *J* = 6.6 Hz, 3H), 0.97 (d, *J* = 7.3 Hz, 3H), 0.94 (d, *J* = 7.3 Hz, 3H). ¹³C NMR (100 MHz, CDCl₃) δ 171.9, 165.7, 145.3, 134.4, 130.5, 129.0 (2C), 128.3 (2C), 118.1, 85.6, 84.6, 75.7, 71.3, 52.6, 47.7, 47.1, 40.2, 36.1, 33.2, 31.3, 31.1, 24.7, 19.2, 18.4, 17.6, 17.1. IR (KBr film) (cm⁻¹) v 3463, 2957, 1710, 1636. HPLC-ESI-HRMS ([M+H]⁺) calcd for C₂₇H₃₈O₅N 456.2745, found 456.2737. |
| 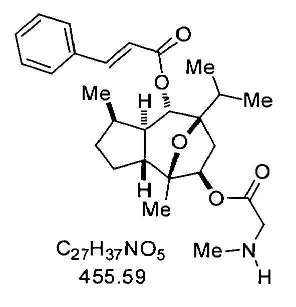 | |
| **42** | ¹H NMR (300 MHz, CDCl₃, 2 diastereomers) δ 7.66 (d, *J* = 16.1 Hz, 1H), 7.52 (m, 2H), 7.39 (m, 3H), 6.39 & 6.38 (2d, *J*= 16.1 Hz, 1H), 5.13 (m, 2H), 4.50 & 4.47 (2dd, *J* = 4.8 & 2.2 Hz, 1H), 4.03 (m, 1H), 3.96 (m, 1H), 2.67 (dd, *J* = 14.6, 8.1 Hz, 1H), 2.28 (m, 1H), 2.13 (sextet, *J* = 6.6 Hz, 1H), 2.06-1.84 (m, 5H), 1.84-1.66 (m, 3H), 1.57 (m, 1H), 1.26 (m, 2H), 1.23 (s, 3H), 1.02 (d, *J*= 6.6 Hz, 3H), 0.97 (d, *J* = 7.0 Hz, 3H), 0.94 (d, *J* = 7.0 Hz, 3H). ¹³C NMR (100 MHz, CDCl₃, 2 diastereomers) δ 173.3 & 173.2, 165.7, 145.3 & 145.2, 134.4, 130.5, 129.0 (2C), 128.2 (2C), 118.2, 85.6, 84.7 & 84.7, 77.0 & 76.8, 75.6 & 75.5, 71.3, 69.6 & 69.5, 47.7, 47.1, 40.2, 33.1 & 33.0, 31.3, 31.1, 30.5 & 30.4, 25.4, 24.7, 19.2, 18.3, 17.6, 17.1. HPLC-ESI-HRMS ([M+H]⁺) calcd for C₂₉H₃₉O₆ 483.2741, found 483.2734;([M+Na]⁺) calcd for C₂₉H₃₈O₆Na 505.2561, found 505.2552. |
| 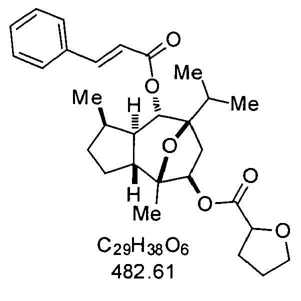 | |
| **43** | [α]_{D}²⁰ -13.8° (c 1.48, CHCl₃). ¹H NMR (400 MHz, CDCl₃) δ 7.67 (d, *J* = 16.1 Hz, 1H), 7.61 (m, 1H), 7.53 (m, 2H), 7.39 (m, 3H), 7.20 (dd, *J* = 3.5, 0.8 Hz, 1H), 6.52 (dd, *J* = 3.5, 1.8 Hz, 1H), 6.40 (d, *J* = 16.1 Hz, 1H), 5.30 (dd, *J* = 7.8, 2.8 Hz, 1H), 5.16 (d, *J* = 10.3 Hz, 1H), 2.75 (dd, *J* = 14.6, 7.8 Hz, 1H), 2.16 (sextet, *J* = 7.0 Hz, 1H), 2.02-1.87 (m, 3H), 1.78 (m, 2H), 1.62 (m, 1H), 1.30 (s, 3H), 1.29 (m, 2H), 1.05 (d, *J* = 6.8 Hz, 3H), 0.99 (d, *J* = 7.0 Hz, 3H), 0.96 (d, *J* = 7.0 Hz, 3H). ¹³C NMR (100 MHz, CDCl₃) δ 165.8, 158.5, 146.7, 145.3, 144.7, 134.4, 130.5, 129.0 (2C), 128.3 (2C), 118.2, 118.1, 112.0, 85.7, 84.9, 75.8, 71.4, 47.7, 47.1, 40.4, 33.3, 31.3, 31.1, 24.8, 19.3, 18.4, 17.6, 17.1. IR (KBr film) (cm⁻¹) v 2956, 1711, 1636. HPLC-ESI- HRMS ([M+H]⁺) calcd for C₂₉H₃₅O₆ 479.2428, found 479.2419. |
| 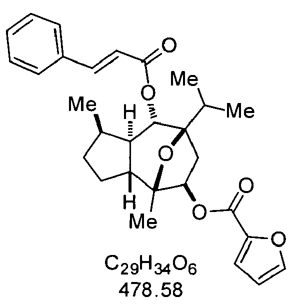 | |
| **44** | [α]_{D}²⁰ -31.5° (c 1.11, CHCl₃). ¹H NMR (500 MHz, CDCl₃) δ 8.80 (d, *J =* 3.8 Hz, 1H), 8.10 (d, *J =* 7.7 Hz, 1H), 7.85 (td, *J =* 7.7, 1.7 Hz, 1H), 7.68 (d, *J* = 16.1 Hz, 1H), 7.53 (dd, *J* = 6.9, 2.7 Hz, 2H), 7.49 (dd, *J*= 7.1, 5.6 Hz, 1H), 7.39-7.37 (m, 3H), 6.41 (d, *J*= 16.1 Hz, 1H), 5.41 (dd, *J* = 8.0, 3.1 Hz, 1H), 5.18 (d, *J* = 10.3 Hz, 1H), 2.82 (dd, *J =* 14.5, 7.7 Hz, 1H), 2.17 (sextet, *J* = 6.8 Hz, 1H), 2.01-1.93 (m, 3H), 1.87-1.82 (m, 1H), 1.80-1.74 (m, 1H), 1.69-1.64 (m, 2H), 1.34 (s, 3H), 1.29-1.24 (m, 1H), 1.06 (d, *J*= 6.9 Hz, 3H), 0.99 (d, *J* = 7.3 Hz, 3H), 0.97 (d, *J* = 6.9 Hz, 3H). ¹³C NMR (126 MHz, CDCl₃) δ 165.6, 164.4, 150.2, 148.1, 145.1, 137.0, 134.3, 130.4, 128.9 (2C), 128.1 (2C), 126.9, 124.9, 118.1, 85.6, 84.8, 76.6, 71.3, 47.7, 47.0, 40.3, 33.2, 31.2, 31.0, 24.6, 19.3, 18.3, 17.5, 16.9. IR (KBr film) (cm⁻¹) v 3446, 2961, 2938, 2907, 1732, 1704, 1635. HPLC-ESI-HRMS ([M+H]⁺) calcd for C₃₀H₃₆O₅N 490.2588, found 490.2580; HPLC-ESI-HRMS ([M+Na]⁺) calcd for C₃₀H₃₅O₅NNa 512.2407, found 512.2398. |
| 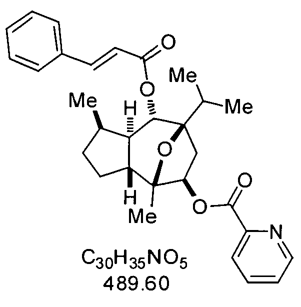 | |

### BIOLOGICAL TESTS

### Generalities

A498 cells were purchased from German Collection of Microorganisms and Cell Cultures, Germany. RC-124 cell line was purchased from CLS, Germany. Minimum Essential Medium (MEM), McCoy's 5a and Dulbecco's Modified Eagle's Medium (DMEM) were purchased from PAA, Austria. Fetal calf serum was purchased from Invitrogen, Germany. Non-essential amino acids and sodium pyruvate were purchased from Sigma-Aldrich, Germany.

### Cell lines

The human kidney carcinoma cell line A498 was cultured in Minimum Essential Medium (MEM, with Earl's salts). The African green monkey cell line BSC-1 and the human embryonic kidney cell line HEK293 were maintained in Dulbecco's Modified Eagle's Medium (DMEM). The human kidney cell line RC-124 was cultured in McCoy's 5a medium. All media were supplemented with 10 % fetal calf serum and penicillin and streptomycin. The cells were maintained at 37°C in a 5 % CO₂ humidified atmosphere. Cells were discarded after 20 passages.

### Cell proliferation assay

Cell proliferation was determined as a function of the metabolic activity by means of the cell proliferation reagent WST-1 (Roche, Mannheim Germany). 4000 cells were seeded in quadruplicates into the wells of 96 well plates and were allowed to attach for 24 hours prior to treatment with different compound concentrations for 48 hours. All samples contained 0.1 % DMSO. After the treatment, the WST-1 reagent was added to the cells according to the manufacturer's protocol. The absorbance was measured with the Inifinite^{®} M200 plate reader (Tecan, Austria) at 450/690 nm. Mean values (n=4) were used to generate dose-response curves by fitting to a four-parameter IC₅₀ equation. The IC₅₀ values for each compound were calculated with the software GraFit 5.0.

The following IC₅₀ values were obtained for the human kidney carcinoma cell line A498. Very similar IC₅₀ values were obtained for the African green monkey cell line BSC-1, the human embryonic kidney cell line HEK293 and the human kidney cell line RC-124.

| **Comp. No.** | **IC₅₀** | | **Comp. No.** | **IC₅₀** | | **Comp. No.** | **IC₅₀** |
|---|---|---|---|---|---|---|---|
| 13 | + | | 19 | + | | 30 | + |
| 14 | + | | 23 | + | | 31 | ++ |
| 15 | ++++ | | 24 | +++ | | 36 | + |
| 16 | ++++ | | 26 | + | | 37 | ++ |
| 17 | ++++ | | 29 | + | | 38 | + |
| 18 | + | | | | | 39 | + |

| | | | | | | | |
|---|---|---|---|---|---|---|---|
| ++++: IC₅₀ < 30 nM +++: 30 nM ≤ IC₅₀ < 100 nM ++: 100 nM ≤ IC₅₀ < 500 nM +: 0.5 µM ≤ IC₅₀ < 5 µM | | | | | | | |

## Claims

1. Compound having the general formula (I): 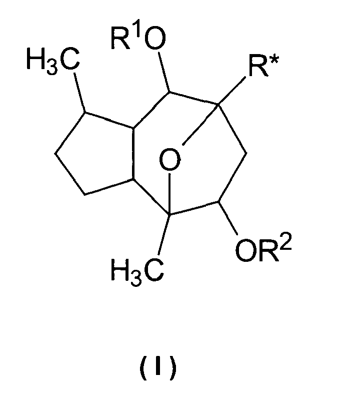 wherein
**R*** represents -CH₃, -C₂H₅, -C₃H₇, -CH(CH₃)₂, cyclo-C₃H₅, cyclo-C₄H₇, cyclo-C₅H₉, cyclo-C₆H₁₁, -Ph, -CH₂-Ph, -CH=CH-Ph;
**R¹** represents -CO-R³, -CO-CH=CH-R³, -CO-CH=C(CH3)-R³,
**R²** represents -CO-CH₂-O-CO-NH-R⁴, -CO-CH₂-O-CO-N(R⁴R^{4*}), -CO-CH₂-OR⁴, -CO-CH₂-O-CO-R⁴, -CO-CH₂-O-CO-O-R⁴, -CO-CH₂-SR⁴, -CO-CH₂-N(R⁴R^{4*}), -CO-CH₂-NH-CO-O-R⁴ -CO-(CH₂)ₙ-OR⁴, -CO-(CH₂)ₙ-R⁴, 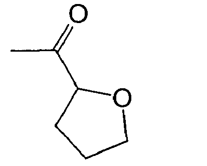 n is an integer selected from 1, 2, 3, 4, 5;
**R³** represents one of the following residues: 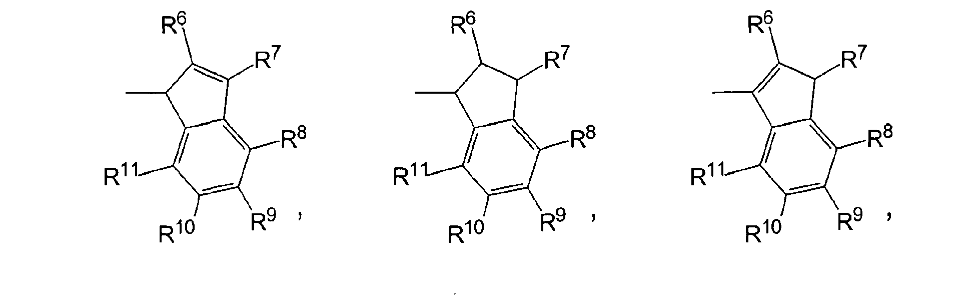 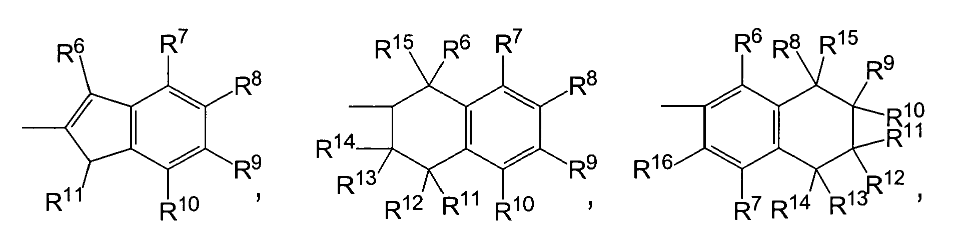 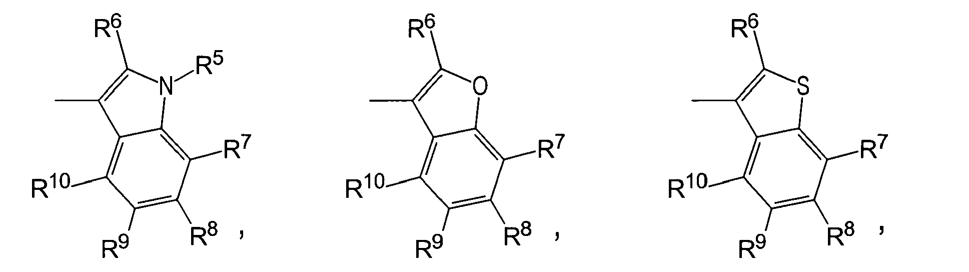 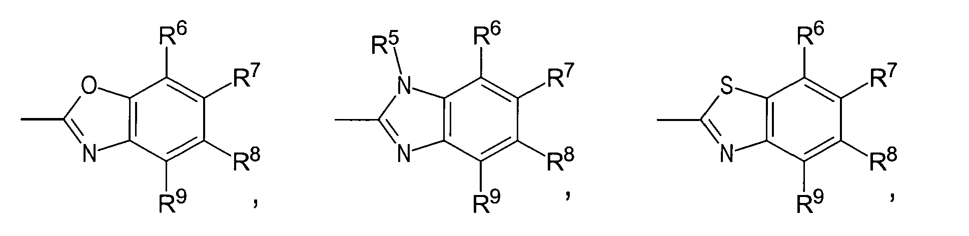 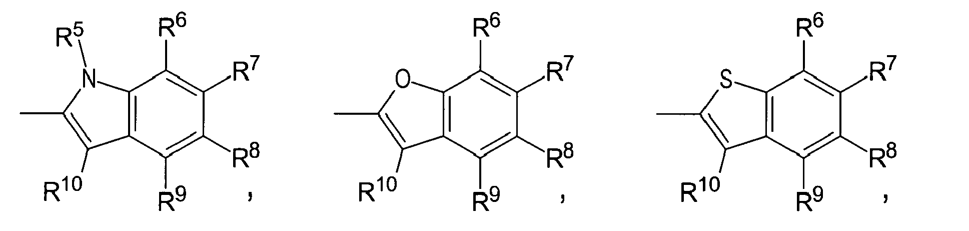 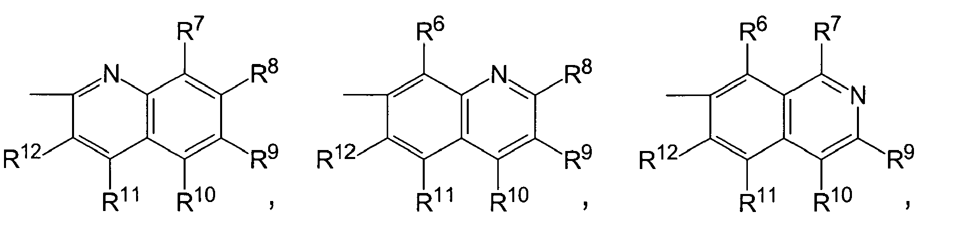 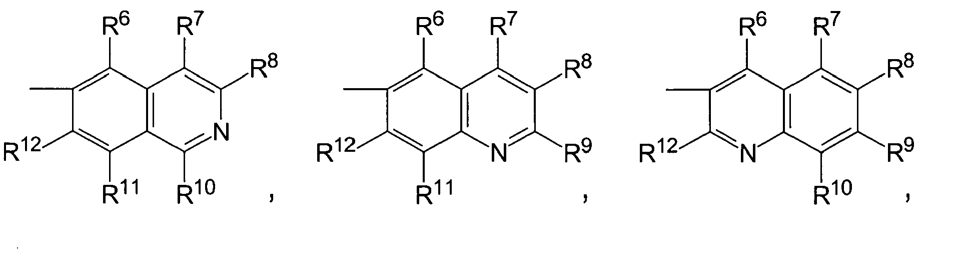 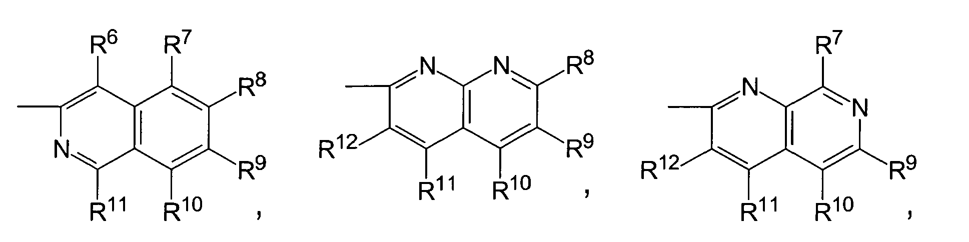 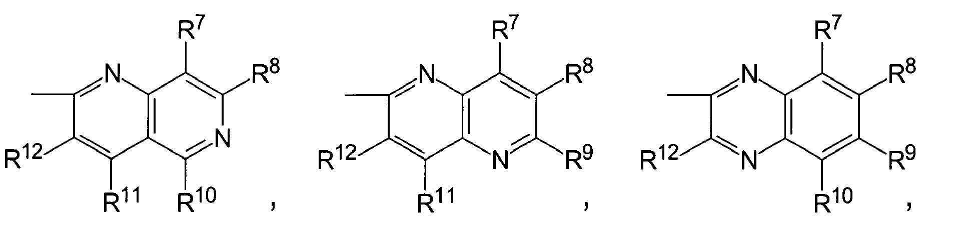 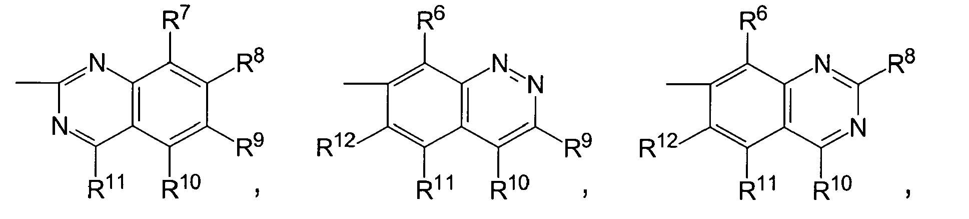 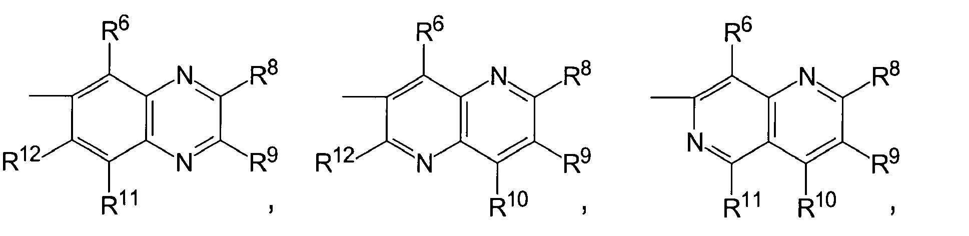 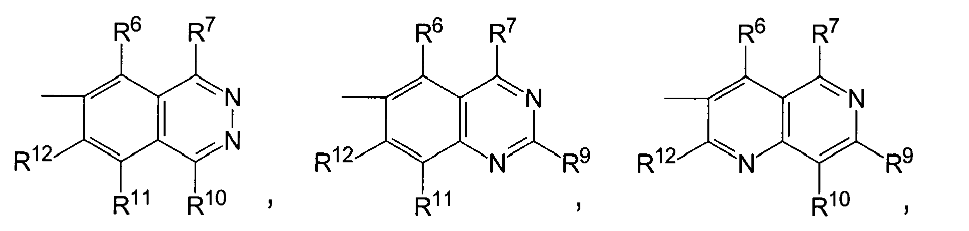 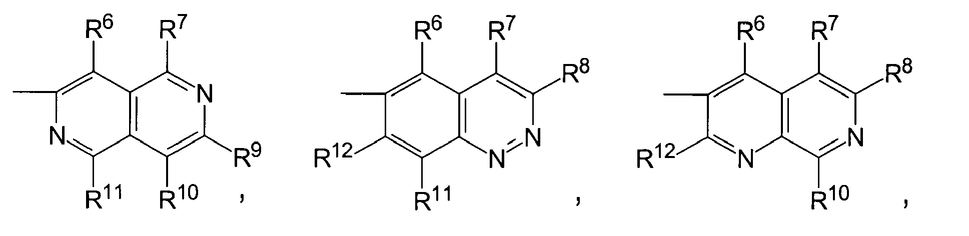 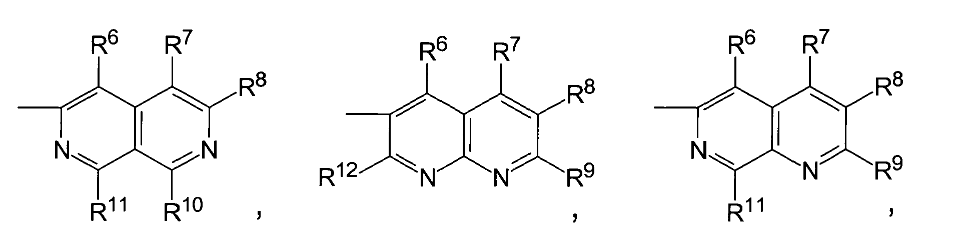 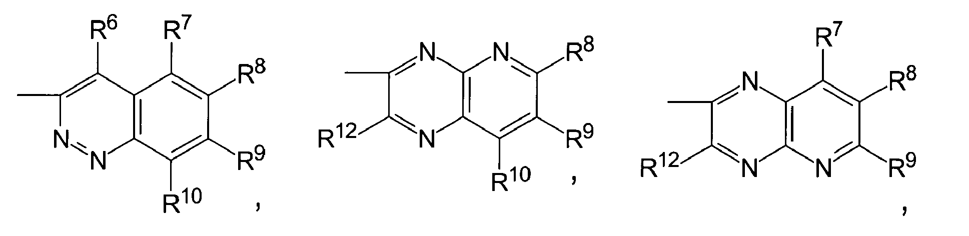 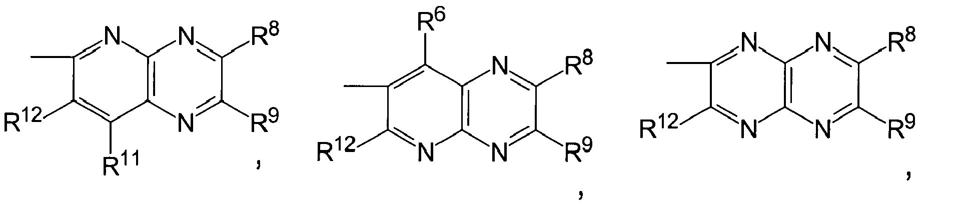 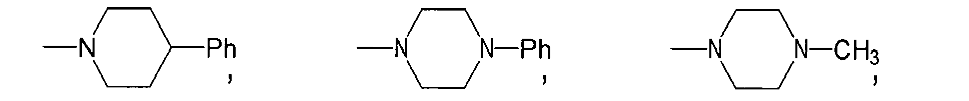 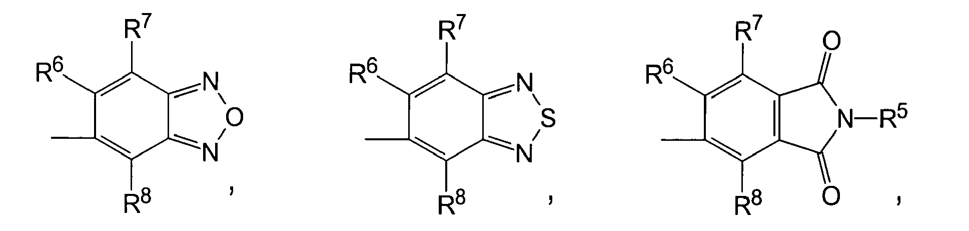 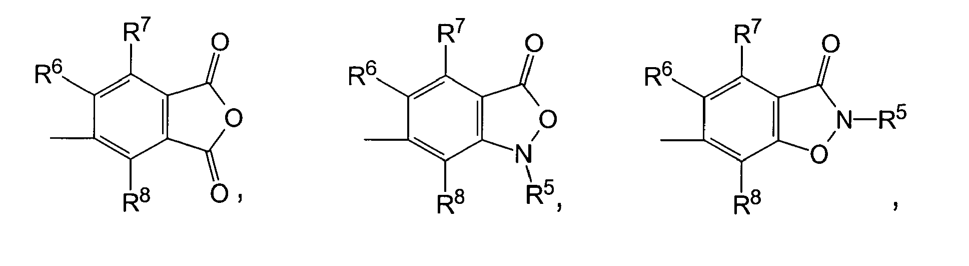 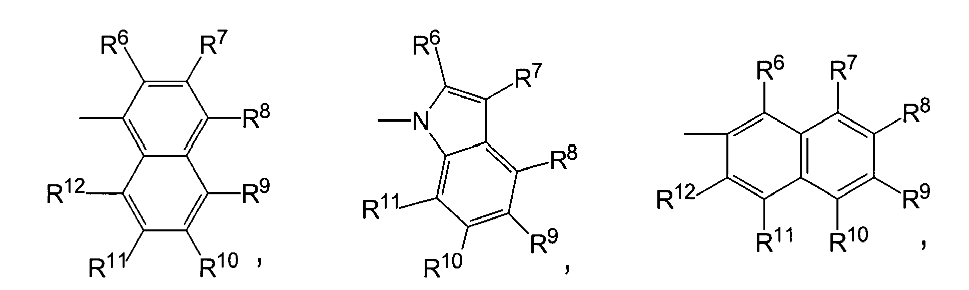 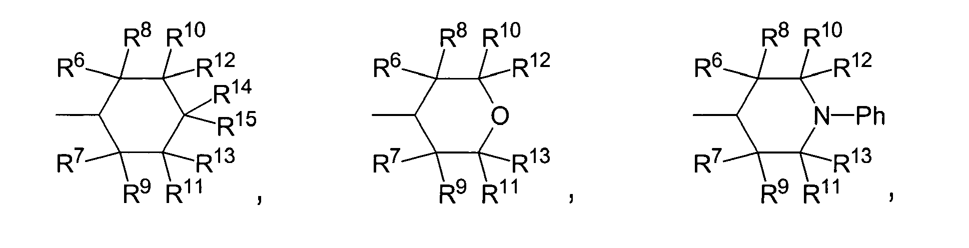 **R⁴, R⁴*, R⁵** represent independently of each other **-R¹⁶, -R¹⁷, -R¹⁸**, -H, -CH₃, -C₂H₅, -C₃H₇, -CH(CH₃)₂, -C₄H₉, -C₅H₁₁, -CH₂-CH(CH₃)₂, -CH(CH₃)-C₂H₅, -C(CH₃)₃, -C₆H₁₃, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₇H₁₅, -C₈H₁₇, -C₉H₁₉, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH2-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂. -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -CH=CH-Ph, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃;
**R⁶** to **R¹⁸** represent independently of each other
-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-Cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CON[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃;
and epimers, enantiomers, stereoisomeric forms, mixtures of enantiomers, diastereomers, mixtures of diastereomers, hydrates, solvates and racemates of the above mentioned compounds and pharmaceutically acceptable salts thereof.

2. Compound according to claim 1, having the general formula (B) 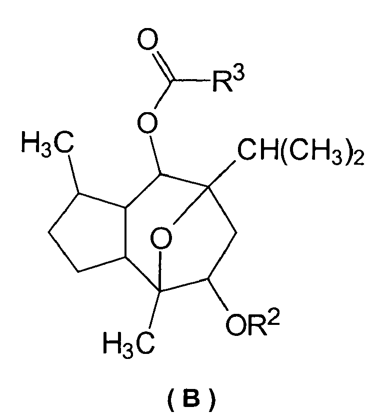 wherein
**R²** and **R³** have the meanings as disclosed in claim 1.

3. Compound according to claim 2, wherein R³ represents 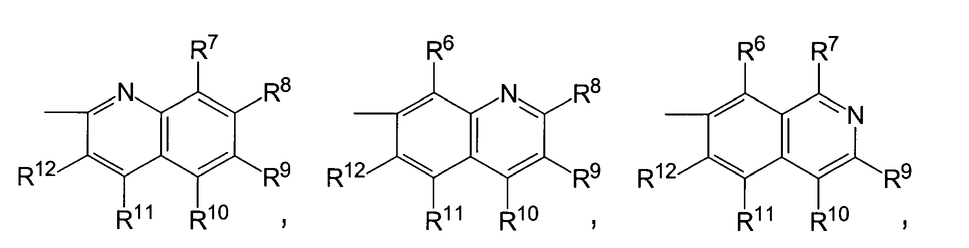 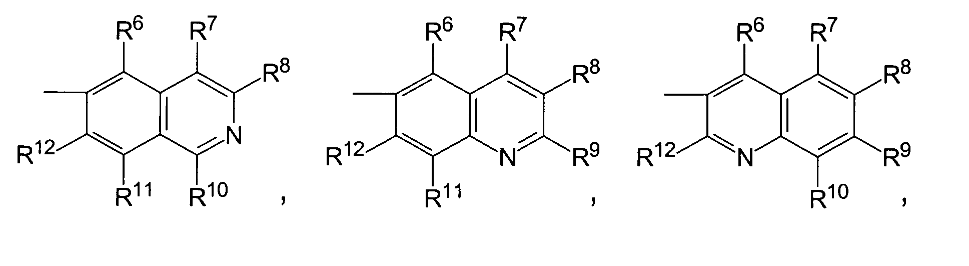 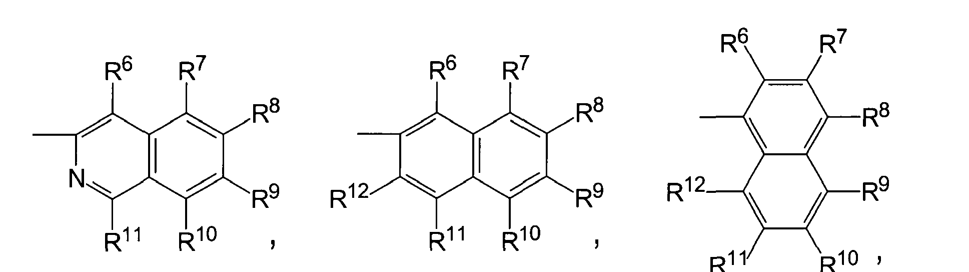 and R⁶ to R¹² has the meanings as disclosed in claim 1.

4. Compound according to claim 1, wherein R¹ represents -CO-CH=CH-R³ or -CO-CH=C(CH₃)-R³ and R³ represents 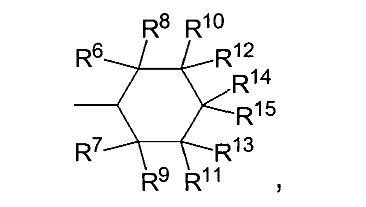 wherein R⁶ to R¹⁵ have the meanings as disclosed in claim 1.

5. Compound according to claim 1, wherein R² represents -CO-CH₂-OR⁴ and R⁴ represents -C₃H₇, -CH=CH₂, -CH₂-CH=CH₂, -CH=CH-CH₃, -C₂H₄-CH=CH₂, -CH₂-CH=CH-CH₃, -CH=CH-C₂H₅, -CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -C≡C-C≡CH.

6. Compound according to any one of claims 1 to 5 having the stereochemistry as shown in formula (C) 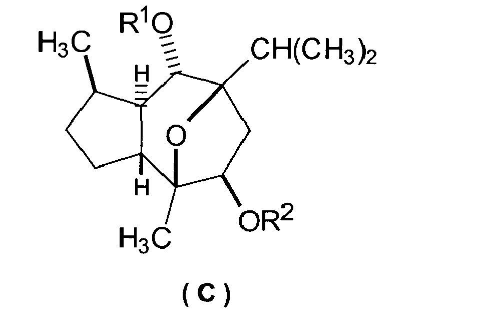 wherein R¹ and R² have the meanings as disclosed in claim 1.

7. Compound according to any one of claims 1 - 6 for use in medicine.

8. Compound according to any one of claims 1 - 6 used or useful for the treatment and/or prophylaxis of cancer.

9. Compound according to any one of claims 1 - 6 used or useful for the treatment and/or prophylaxis of cancer, wherein the cancer is selected from the group consisting of mammal cancer, ovarian cancer, hepatic cancer, leukemia, colon cancer, melanoma, prostate cancer, renal cancer, and lung cancer.

10. Compound according to any of the claims 1 - 6 used or useful for the treatment and/or prophylaxis of renal cancer.

11. Use of a compound according to any one of claims 1 - 6 for the manufacture of a medicament for the treatment and/or prophylaxis of cancer.

12. Pharmaceutical composition comprising at least one compound according to any of the claims 1 - 6 as an active ingredient, together with at least one pharmaceutically acceptable carrier, cryprotectant, lyoprotectant, excipient and/or diluent.

13. Pharmaceutical composition according to claim 12, wherein the pharmaceutical composition is in the form of a lyophilisate, a sustained release dosage form, an oral formulation, an injection formulation, or a liquid buffer solution.

14. Pharmaceutical composition according to claim 12 or 13 suitable for intravenous administration, oral administration, or for administration by inhalation.
